# EUROPEAN PATENT APPLICATION

(11) **EP 1 535 637 A1**
(43) Date of publication of application: **01.06.2005**
(21) Application number: 04104421.5
(22) Date of filing: 14.09.2004
(51) Int. Cl.: A61M 5/14, A61M 5/172

(54) **Automatic dose infusion apparatus**

(30) Priority: 28.11.2003 KR 2003085497; 28.11.2003 KR 2003085498; 03.12.2003 KR 2003087263
(71) Applicant: Woo Young Medical Co., Ltd., Paju-City, Kyunggi-Do (KR)
(72) Inventor: Lee, Yang Soo, Goyang-City, Kyunggi-Do (KR); Kim, Sang Hwa, Dongdaemun-Gu, Seoul (KR); Lee, Jong Hee, Seoul (KR)
(74) Representative: Gervasi, Gemma, Dr.

(57) **Abstract**

An automatic dose infusion apparatus (2) that is capable of electronically controlling a medicinal substance storage chamber (6) and a control chamber (8), electronically regulating infusion of the medicinal substance, and constantly maintaining a prescribed amount of medicinal substance on the basis of the number of rotations of a motor so that a patient can easily operate the automatic dose infusion apparatus so as to automatically infuse a dose of medicinal substance into himself/herself without regard to time and situation, and a method of controlling the same. The automatic dose infusion apparatus comprises a housing having three compartments defined therein, a medicinal substance storage chamber (6) formed in the upper compartment of the housing for storing a medicinal substance pack, a control chamber (8) formed in the middle compartment of the housing while being disposed below the medicinal substance storage chamber.

## Description

### BACKGROUND OF THE INVENTION

### Field of the Invention

The present invention relates generally to an automatic dose infusion apparatus, and more particularly to an automatic dose infusion apparatus that is capable of electronically controlling a medicinal substance storage chamber and a control chamber, electronically regulating infusion of the medicinal substance, and constantly maintaining a prescribed amount of medicinal substance on the basis of the number of rotations of a motor so that a patient can easily operate the automatic dose infusion apparatus so as to automatically infuse a dose of medicinal substance into himself/herself without regard to time and situation. Also, the present invention relates to a method of controlling such an automatic dose infusion apparatus as described above.

### Description of the Related Art

In the past, a doctor prescribed a dose of medicinal substance to be infused into a patient. A nurse securely placed a container containing the medicinal substance at a position higher than the region of the patient where an injection of the medicinal substance is to be given, and then controls, on the basis of her experience, a valve having a twisted screw-type opening and closing unit mounted therein to infuse the prescribed amount of medicinal substance.

In the case that a dose of medicinal substance, such as an analgesic, is to be infused into a patient, it is necessary to temporarily increase the dose of medicinal substance because the patient may experience intermittent convulsions or pains. However, it is very dangerous for the patient to adjust the dose of medicinal substance on his/her own. Consequently, a nurse adjusts the dose of medicinal substance only after a doctor writes a prescription, which requires substantial time. Also, the doctor or the nurse may waste his/her time.

In the case that a nurse who adjusts the dose of medicinal substance has little experience, it is very difficult for the nurse to adjust the dose of medicinal substance every hour so that the prescribed amount of medicinal substance can be accurately infused into a patient. In the case that a patient is given an injection only when he/she is affected by a specific disease while being not normally injected, i.e., in the case that the patient has symptoms of acute cardiac arrest, it is necessary that he/she always carry medicinal substance because rapid injection of the medicinal substance is required and so that the accurate amount of medicinal substance be injected whenever the patient is to be given an injection. Consequently, it is necessary that the patient be skilled at self-administration of the medicinal substance.

Even though the patient is skilled at self-administration of the medicinal substance, there may be a possibility that the medicinal substance is excessively injected by accident. Furthermore, the medicinal substance may contain a foreign material or air therein, or a tube for supplying the medicinal substance may be squeezed, which puts the patient in danger.

### SUMMARY OF THE INVENTION

Therefore, the present invention has been made in view of the above problems, and it is an object of the present invention to provide an automatic dose infusion apparatus that is capable of electronically controlling a medicinal substance storage chamber and a control chamber, electronically regulating infusion of the medicinal substance, and constantly maintaining a prescribed amount of medicinal substance on the basis of the number of rotations of a motor so that a patient can easily operate the automatic dose infusion apparatus so as to automatically infuse a dose of medicinal substance into himself/herself without regard to time and situation.

It is another object of the present invention to provide an automatic dose infusion apparatus that is capable of finely and accurately performing a controlling operation according to various prescribed algorithms through the use of a door-detecting sensor, an air-detecting sensor, a block-detecting sensor, and a rotation-detecting sensor for detecting the number of rotations of a motor so that infusion of medicinal substance is optimized while a prescribed amount of medicinal substance is automatically infused, and the medicinal substance is infused while the highest safety is guaranteed, whereby a patient can safely and easily self-inject the prescribed amount of medicinal substance.

It is yet another object of the present invention to provide a method of controlling such an automatic dose infusion apparatus as described above.

In accordance with one aspect of the present invention, the above and other objects can be accomplished by the provision of an automatic dose infusion apparatus, comprising: a housing having three compartments defined therein; a medicinal substance storage chamber formed in the upper compartment of the housing for storing a medicinal substance pack; a control chamber formed in the middle compartment of the housing while being disposed below the medicinal substance storage chamber, the control chamber including a button part for setting the infusion amount of medicinal substance and time intervals, an LCD for displaying setup information, and a circuit board with various circuit elements mounted thereto; a driving chamber formed in the lower compartment of the housing while being disposed below the control chamber, the driving chamber including a pressure pump mounted therein for pumping the medicinal substance so that the medicinal substance can be discharged through a tube, the driving chamber serving to regulate the infusion of the medicinal substance by controlling the operation of the pressure pump; a bolus switch connected to a prescribed position of one side of the housing via an electric cord for generating an additional medicinal substance infusion command signal; and a tube extending from the inside of the driving chamber to the outside of the driving chamber for delivering the medicinal substance discharged from the medicinal substance pack to a needle.

Preferably, the driving chamber includes: a guide groove formed at the top of the housing for allowing the tube to be securely located therein; a hinged cover attached to the top of the housing for covering the guide groove and the pressure pump; and a check valve location groove formed at the top of the housing for allowing a check valve, formed at a prescribed position of the middle of the tube for regulating infusion of the medicinal substance, to be securely located therein.

Preferably, the hinged cover includes: a supporting bar formed at the lower surface of the hinged cover, such that the supporting bar extends a prescribed length in the longitudinal direction of the hinged cover, for pressing the tube against the pressure pump; and a pressing protrusion for pressing the check valve so that the medicinal substance can be infused.

More preferably, the pressure pump includes: a plurality of pressing pieces disposed in a groove formed at the top of the housing, each of the pressing pieces being formed in the shape of a "U" such that the tube can be securely located in the upper part of the pressing piece and having a through-hole formed at the middle thereof; a cam mounted in the through-holes of the pressing pieces, the cam having a screw formed on the outer circumference thereof; a motor for transmitting a driving force to the cam; and a spring for connecting a camshaft of the cam and a motor shaft of the motor such that the driving force of the motor can be accurately transmitted to the cam even when the camshaft and the motor shaft are not arranged in a straight line.

More preferably, the pressing pieces are arranged such that one of the pressing pieces is connected to a neighboring pressing piece by means of the cam, the pressing pieces being moved vertically in the form of a sine wave to press the tube when the cam is driven so that the medicinal substance can be delivered.

Preferably, the check valve comprises: a valve housing having one end connected to a main tube for delivering the medicinal substance from the inside of the apparatus and the other end connected to a discharging tube for discharging the medicinal substance to the outside of the apparatus; and an opening and closing member mounted in the valve housing such that the opening and closing member is disposed at a middle space part defined in the valve housing, the opening and closing member being formed in the shape of a taper so that the opening and closing member can be moved vertically to open or close a flow channel defined in the valve housing.

In accordance with another aspect of the present invention, there is provided an automatic dose infusion apparatus, comprising: a housing having three compartments defined therein; a medicinal substance storage chamber formed in the upper compartment of the housing for storing a medicinal substance pack; a driving chamber for regulating infusion of the medicinal substance, the driving chamber including a pressure pump mounted therein for pumping the medicinal substance so that the medicinal substance can be discharged through a tube; a bolus switch connected to a prescribed position of one side of the housing via an electric cord for generating an additional medicinal substance infusion command signal; a tube extending from the inside of the driving chamber to the outside of the driving chamber for delivering the medicinal substance discharged from the medicinal substance pack to a needle; and a check valve including a valve housing having one end connected to one middle end of the tube and the other end connected to the other middle end of the tube, and an opening and closing member mounted in the valve housing such that the opening and closing member can be moved vertically to regulate infusion of the medicinal substance, wherein the automatic dose infusion apparatus further comprises: a membrane attached to a lower supporting surface of the check valve such that the membrane can be expanded or contracted by the pressure inside the valve housing; a block-detecting sensor disposed below the membrane for detecting blockage of the tube, the block-detecting sensor generating a high signal when the membrane is expanded; a door-detecting sensor mounted to the inner wall of the housing for detecting whether the hinged cover is closed or not, the door-detecting sensor generating a high signal when the door is closed; an air-detecting sensor disposed below the tube for detecting whether or not air is contained in the medicinal substance passing through the tube, the air-detecting sensor generating a high signal when only medicinal substance passes through the tube and the air-detecting sensor generating a low signal when medicinal substance containing air therein passes through the tube; and a control unit for performing a control operation so that the operation of the pressure pump is stopped when a signal detected by means of the block-detecting sensor, the door-detecting sensor, or the air-detecting sensor is abnormal.

Preferably, the block-detecting sensor, the door-detecting sensor, and the air-detecting sensor are proximity sensors each for generating a high signal when the sensor approaches liquid, metal, or skin.

Preferably, the automatic dose infusion apparatus further comprises: a rotation-detecting sensor mounted in the driving chamber for detecting the number of rotations of a motor that drives the pressure pump.

More preferably, the automatic dose infusion apparatus further comprises: a spring connected to the motor, the spring being provided at one end thereof with a protruded rotary part, to which a magnet is attached, and the rotation-detecting sensor is a hall sensor for detecting rotation of a motor shaft of the motor.

In accordance with another aspect of the present invention, there is provided an automatic dose infusion apparatus, comprising: a button unit including a mode selection button for selecting an injection mode so that a prescribed amount of medicinal substance can be automatically infused into a patient, a start/stop button for starting or stopping infusion of the medicinal substance, and an adjustment button for increasing or decreasing the infusion amount of the medicinal substance; a bolus switch for performing additional self-infusion of the medicinal substance in a patient controlled analgesia (PCA) mode; a door-detecting sensor for detecting whether the cover that securely locates a tube and accommodates the tube is closed or not; an air-detecting sensor for detecting whether air is contained in the tube or not; a block-detecting sensor for detecting whether the tube is blocked or not; a motor for actuating a pressure pump, and a motor-driving unit for driving the motor; an LCD for displaying information, such as the mode selected by means of the button unit, the adjusted amount of the medicinal substance, and infusion start/stop status, so that the patient can easily recognize the information; and an LCD-driving unit for driving the LCD, wherein the automatic dose infusion apparatus further comprises: an encoder for detecting the number of rotations of the motor; a buzzer for informing an abnormality of infusion of the medicinal substance, and a buzzer-driving unit for driving the buzzer; a data storage unit for storing medicinal substance infusion algorithms according to a continuous infusion mode, a patient controlled analgesia (PCA) mode, an intermittent infusion mode, a total parenteral nutrition (TPN) infusion mode, and a multi-step infusion mode; and a control unit for receiving a signal according to the input of each button of the button unit to perform an algorithm according to the corresponding mode previously stored in the data storage unit, detecting the number of rotations of the motor in the infusion mode to control the operation of the motor, receiving the detected signal from each sensor to determine whether an abnormality is present or not, stopping the operation of the motor and operating the buzzer when the abnormality is present, so that infusion of the medicinal substance can be safely and accurately performed.

In accordance with yet another aspect of the present invention, there is provided a method of controlling an automatic dose infusion apparatus as described above, comprising the steps of: applying electric power to the automatic dose infusion apparatus; performing an initial self-test; performing an initial mode; determining whether a prescribed mode is selected or not; selecting an infusion mode; preparing for the infusion mode; performing infusion of medicinal substance; informing that the infusion of medicinal substance will be stopped in approximately 10 minutes; generating a stop code; resetting flow rate of the medicinal substance during the operation of the automatic dose infusion apparatus; checking an error in connection with the operation of a motor; and stopping the infusion of the medicinal substance.

Preferably, the infusion mode comprises: a continuous infusion mode for continuously infusing a prescribed amount of medicinal substance from the time when infusion of the medicinal substance is initiated to the time when infusion of the medicinal substance is completed; a patient controlled analgesia (PCA) mode for further infusing another prescribed amount of medicinal substance in addition to the prescribed amount of the medicinal substance in the continuous infusion mode; an intermittent infusion mode for presetting periods of infusion time and interruption time from the time when infusion of the medicinal substance is initiated to the time when infusion of the medicinal substance is completed, and resetting the infusion amount of the medicinal substance at each period of infusion time so that the infusion and the interruption of the medicinal substance can be alternately performed; a total parenteral nutrition (TPN) infusion mode for gradually increasing the amount of the medicinal substance for a prescribed period of time, infusing a prescribed amount of the medicinal substance for a prescribed period of time, and gradually decreasing the amount of the medicinal substance for a prescribed period of time; and a multi-step infusion mode for irregularly changing the amount of medicinal substance on the basis of multiple time zones so that the irregular amount of the medicinal substance can be infused on the basis of the multiple time zones.

Preferably, the infusion mode selecting step comprises: commonly selecting the total infusion amount of the medicinal substance, the infusion speed of the medicinal substance; further selecting the infusion amount of the medicinal substance when the bolus switch is manipulated and a lock out time in the patient controlled analgesia mode; further selecting an infusion time of the medicinal substance and an infusion stop time of the medicinal substance in the intermittent infusion mode; further selecting a ramp up time of the medicinal substance, and a ramp down time of the medicinal substance in the total parenteral nutrition (TPN) infusion mode; and further selecting an infusion step in the multi-step infusion mode.

### BRIEF DESCRIPTION OF THE DRAWINGS

The above and other objects, features and other advantages of the present invention will be more clearly understood from the following detailed description taken in conjunction with the accompanying drawings, in which:
FIG. 1 is a perspective view showing an automatic dose infusion apparatus according to a preferred embodiment of the present invention;
FIG. 2 is a perspective view showing a pressure pump and a regulating unit of the automatic dose infusion apparatus according to the preferred embodiment of the present invention;
FIG. 3 is a side sectional view of the pressure pump of the automatic dose infusion apparatus according to the preferred embodiment of the present invention;
FIGS. 4a and 4b are views respectively showing a cam of the automatic dose infusion apparatus according to the preferred embodiment of the present invention;
FIG. 5 is a view showing a pumping operation of the automatic dose infusion apparatus according to the preferred embodiment of the present invention;
FIG. 6 is a side sectional view showing the regulating unit of the automatic dose infusion apparatus according to the preferred embodiment of the present invention;
FIGS. 7a and 7b are side sectional views respectively showing a door-detecting sensor of the automatic dose infusion apparatus according to the preferred embodiment of the present invention;
FIGS. 8a and 8b are side sectional views respectively showing an air-detecting sensor of the automatic dose infusion apparatus according to the preferred embodiment of the present invention;
FIGS. 9a and 9b are side sectional views respectively showing a block-detecting sensor of the automatic dose infusion apparatus according to the preferred embodiment of the present invention;
FIG. 10 is a side sectional view showing a rotation-detecting sensor for detecting the number of rotations of a motor of the automatic dose infusion apparatus according to the preferred embodiment of the present invention;
FIGS. 11a to 11e are graphs respectively showing time profiles at each mode of the automatic dose infusion apparatus according to the preferred embodiment of the present invention;
FIG. 12 is a block diagram showing the construction of the automatic dose infusion apparatus according to the preferred embodiment of the present invention;
FIG. 13 is a flow chart showing all the operational algorithms of the automatic dose infusion apparatus according to the preferred embodiment of the present invention;
FIG. 14 is a flow chart showing a setup preparation mode algorithm of the automatic dose infusion apparatus according to the preferred embodiment of the present invention;
FIG. 15 is a flow chart showing a data selection setup algorithm of the automatic dose infusion apparatus according to the preferred embodiment of the present invention;
FIG. 16 is a flow chart showing key inputs for an infusion operation of the automatic dose infusion apparatus according to the preferred embodiment of the present invention;
FIG. 17 is a flow chart showing a self-test algorithm of the automatic dose infusion apparatus according to the preferred embodiment of the present invention;
FIG. 18 is a flow chart showing an algorithm, when the motor is driven, of the automatic dose infusion apparatus according to the preferred embodiment of the present invention;
FIG. 19a is a flow chart showing an algorithm, in a continuous infusion mode, of the automatic dose infusion apparatus according to the preferred embodiment of the present invention;
FIG. 19b is a flow chart showing an algorithm, in a patient controlled analgesia (PCA) mode, of the automatic dose infusion apparatus according to the preferred embodiment of the present invention;
FIG. 19c is a flow chart showing an algorithm, in an intermittent infusion mode, of the automatic dose infusion apparatus according to the preferred embodiment of the present invention;
FIG. 19d is a flow chart showing an algorithm, in a total parenteral nutrition (TPN) infusion mode, of the automatic dose infusion apparatus according to the preferred embodiment of the present invention; and
FIG. 19e is a flow chart showing an algorithm, in a multi-step infusion mode, of the automatic dose infusion apparatus according to the preferred embodiment of the present invention.

### DESCRIPTION OF THE PREFERRED EMBODIMENTS

Now, preferred embodiments of the present invention will be described in detail with reference to the accompanying drawings.

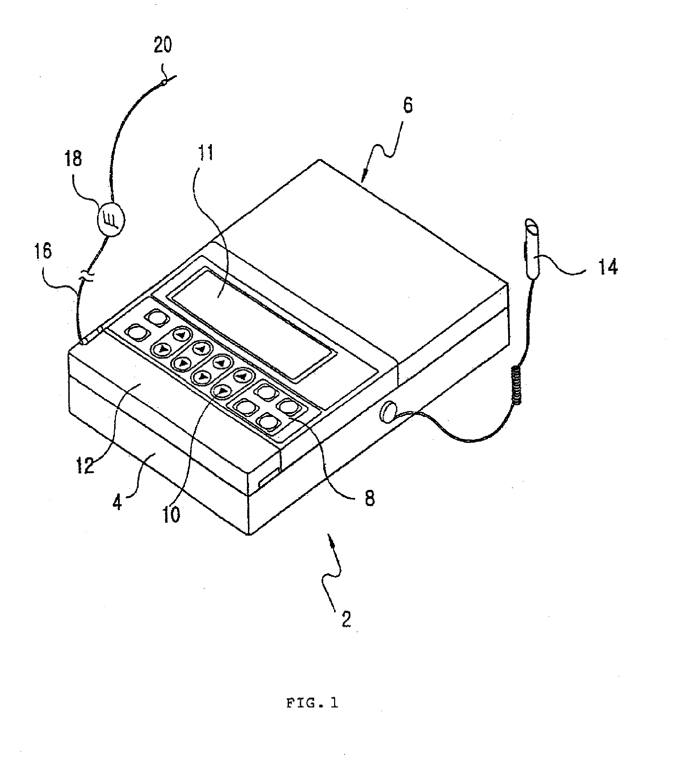

FIG. 1 is a perspective view showing an automatic dose infusion apparatus 2 according to a preferred embodiment of the present invention.

Referring to FIG. 1, the automatic dose infusion apparatus 2 includes a housing 4 forming the external appearance of the apparatus 2. The interior of the housing 4 is divided into three compartments. The upper compartment is a medicinal substance storage chamber 6 for storing medicinal substance packs, and the middle compartment is a control chamber 8 for controlling the infusion amount of medicinal substance and a period of time for infusing the medicinal substance. The control chamber 8 has an LCD 11 for displaying setup status.

The lower compartment is a driving chamber 12 for pumping medicinal substance to discharge the medicinal substance through a discharging tube 16 and regulating infusion of the medicinal substance.

To a prescribed position of one side of the housing 4 is connected a bolus switch 14 via an electric cord so that a user can control the bolus switch 14 to temporarily increase the dose of medicinal substance. On the middle part of a discharging tube 16 connected between a needle 20 and the housing 4 is mounted an air filter 18 for filtering air contained in medicinal substance passing though the discharging tube 16 so that the air is discharged out of the discharging tube 16. On the control chamber 8 is disposed a button unit 10 for performing various setup operations.

Consequently, a patient may carry the automatic dose infusion apparatus 2 at all times so that the patient can easily and conveniently self-inject a prescribed amount of medicinal substance without regard to time and situation. Also, various sensors systematically cooperate with one another to control infusion of medicinal substance, whereby safety of the patient is guaranteed when the medicinal substance is infused. Consequently, no private nurse is necessary for daily life.

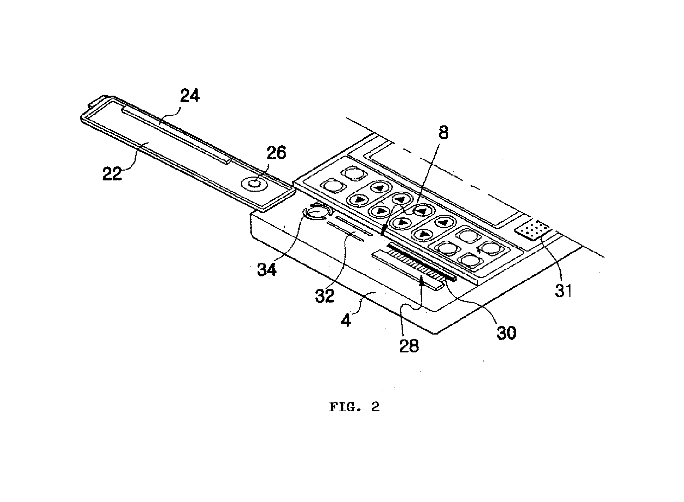

FIG. 2 is a perspective view showing a pressure pump and a regulating unit of the automatic dose infusion apparatus according to the preferred embodiment of the present invention.

Referring to FIG. 2, the driving chamber 12 for generating a driving force so that medicinal substance can be infused when a medicinal substance infusion control signal is applied to the driving chamber 12 is provided at the lower part of the housing 4 of the automatic dose infusion apparatus 2 according to the preferred embodiment of the present invention. To the top of the driving chamber 12 is connected a hinged cover 22. The hinged cover 22 includes a supporting bar 24 formed at the lower surface of the hinged cover 22, such that the supporting bar 24 extends a prescribed length in the longitudinal direction of the hinged cover 22, for pressing the discharging tube 16, and a pressing protrusion 26 for pressing a check valve mounted in the discharging tube 16 so that the check valve can be maintained open.

In the driving chamber is mounted a pressure pump 28. The pressure pump 28 includes a motor (not shown) for generating a rotary driving force with the prescribed number of rotations according to electric control, and a plurality of pressing pieces 30 coupled horizontally with each other such that the pressing pieces 30 can be moved vertically when the rotary driving force from the motor is transmitted to the pressing pieces 30.

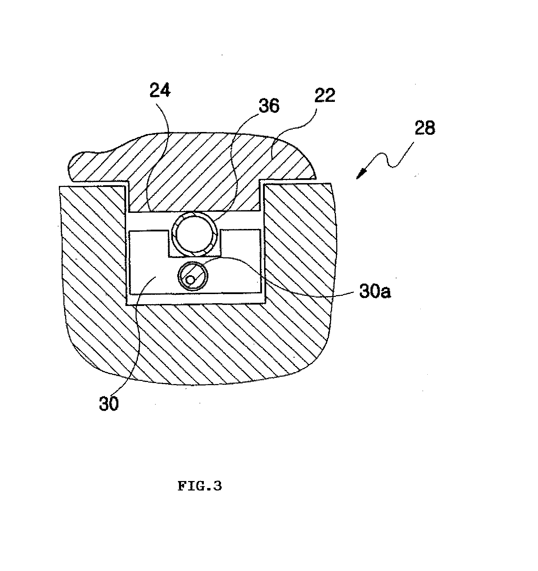

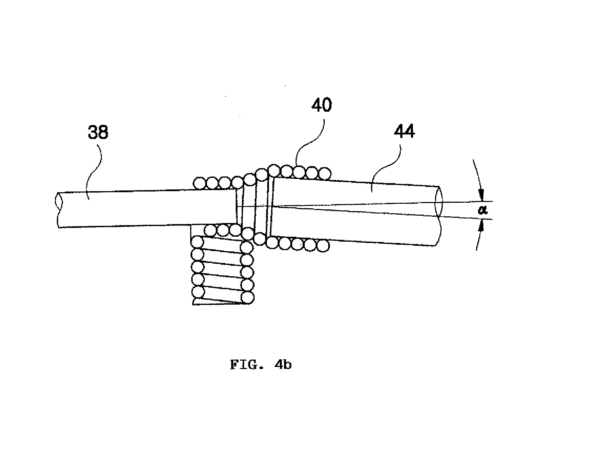

The pressing pieces 30 are moved vertically in the form of a sine wave by means of a rotary force of a cam 42 (Refer to FIG. 4b), which will be described below. Consequently, the pressing pieces 30 press the lower part of a main tube 36 (Refer to FIG. 3), which will be described below, in the form of a sine wave, so that medicinal substance contained in the discharging tube 16 can be moved in one direction.

At the top of the housing 4, to which the hinged cover 22 of the driving chamber 12 is attached, is formed a guide groove 32 for guiding the discharging tube 16 while the discharging tube 16 is securely engaged in the guide groove 32. At the top of the housing 4 is also formed a check valve location groove 34, which is disposed adjacent to the guide groove 32.

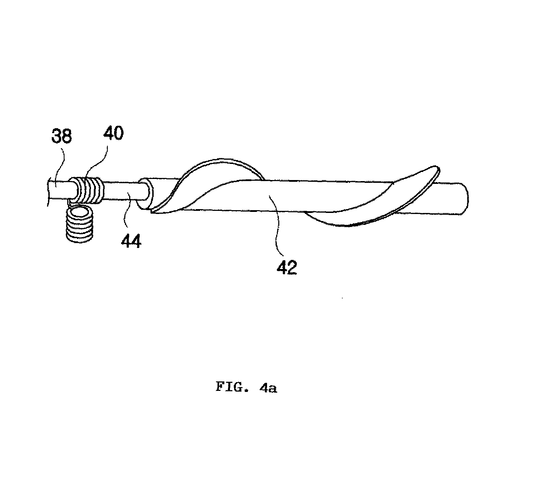

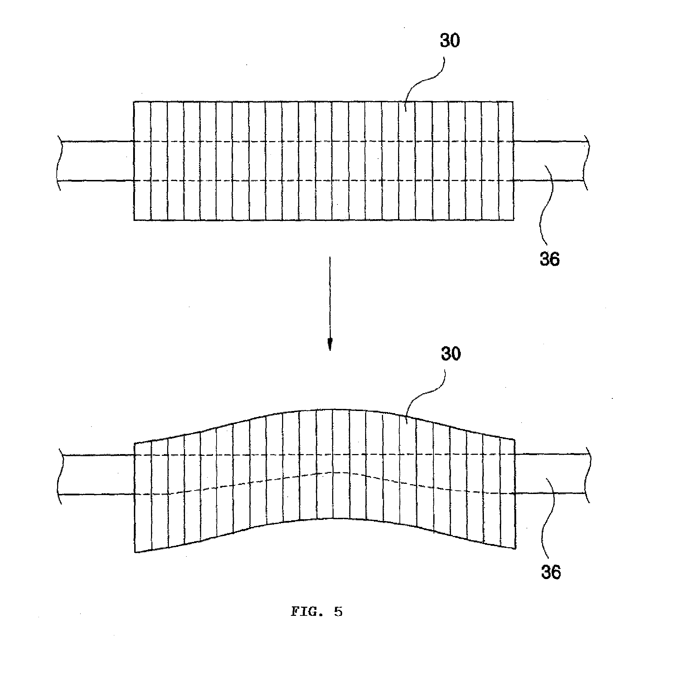

FIG. 3 is a side sectional view of the pressure pump of the automatic dose infusion apparatus according to the preferred embodiment of the present invention, FIGS. 4a and 4b are views respectively showing a cam of the automatic dose infusion apparatus according to the preferred embodiment of the present invention, and FIG. 5 is a view showing a pumping operation of the automatic dose infusion apparatus according to the preferred embodiment of the present invention.

The pressure pump 28 of the automatic dose infusion apparatus 2 according to the preferred embodiment of the present invention has the following construction. As shown in FIG. 3, the plurality of pressing pieces 30 are mounted in a groove formed at the top of the housing 4 while the pressing pieces 30 are arranged adjacent to each other in parallel. Each pressing piece 30 is provided at the middle thereof with a through-hole 30a, in which the cam 42 is mounted. Each pressing piece 30 is formed in the shape of a "U". Consequently, the main tube 36 is securely located in a groove formed at the upper part of the pressing piece 30. The upper surface of the main tube 35 is constantly pressed by means of the supporting bar 24 formed at the lower surface of the hinged cover 22.

The cam 42 has a camshaft 44, which is connected to a motor shaft 38 of the motor (not shown) that generates a rotary driving force. The cam 42 is formed in the shape of a screw, and the cam 42 is mounted longitudinally in the through-holes 30a of the pressing pieces 30. Consequently, as the cam 42 is rotated by means of the motor, the pressing pieces 30 are moved vertically in cooperation with the cam 42. In other words, the pressing pieces 30 are moved vertically in the form of a sine wave.

The camshaft 44 and the motor shaft 38 are connected with each other by means of a connecting member, such as a spring 40, as shown in FIGS. 4a and 4b. Specifically, one end of the spring 40 is fixedly fitted on the camshaft 44 and the other end of the spring 40 is fixedly fitted on the motor shaft 38. Consequently, the rotary force of the motor can be accurately transmitted to the cam 42 even when the camshaft 44 and the motor shaft 38 are not arranged in a straight line as shown in FIG. 4b.

As the cam 42 is rotated by means of the motor, the pressing pieces 30 are moved vertically in cooperation with the cam 42 in the form of a sine wave as shown in FIG. 5 so that medicinal substance contained in the main tube 36 can be moved in one direction.

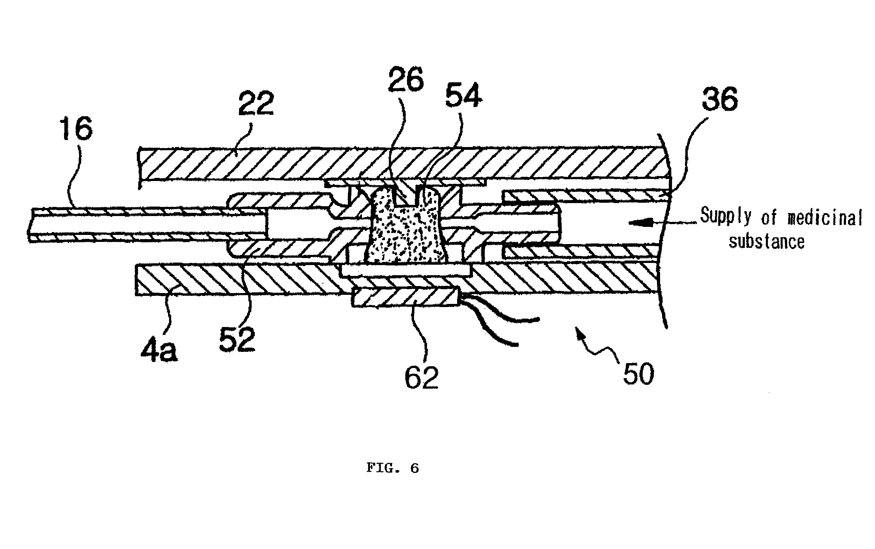

FIG. 6 is a side sectional view showing the regulating unit of the automatic dose infusion apparatus according to the preferred embodiment of the present invention.

Referring to FIG. 6, the regulating unit of the automatic dose infusion apparatus 2 according to the preferred embodiment of the present invention is a check valve 50. The check valve 50 includes a valve housing 52 for regulating flow of medicinal substance. The valve housing 52 has one end connected to the main tube 36 and the other end connected to the discharging tube 16. The valve housing 52 is pressed by means of the pressing protrusion 26 while the valve housing 52 is securely located in the check valve location groove 34 (Refer to FIG. 2). Through the valve housing 52 is formed a flow channel, which extends in the longitudinal direction of the housing 52.

In the valve housing 52 is mounted an opening and closing member 54 made of silicon packing. The opening and closing member 54 is formed in the shape of a taper so that the opening and closing member 54 can be moved vertically to open or close the flow channel. Medicinal substance is introduced into the valve housing 52 through the main tube 36, passes below the opening and closing member, and is then discharged through the discharging tube 16. If the pressing protrusion 26 does not press the top of the opening and closing member 54 when medicinal substance is introduced into the valve housing 52 through the main tube 36 connected to a medicinal substance pack (not shown), the medicinal substance introduced into the valve housing 52 pushes the opening and closing member 54 upward to intercept the flow channel. Consequently, the medicinal substance is not moved from the main tube 36 to the discharging tube 16.

If the pressing protrusion 26 presses the top of the opening and closing member 54 as the hinged cover 4 is attached to the housing 4, on the other hand, the opening and closing member 54 is pressed downward. As a result, a gap is created between the flow channel of the valve housing 52 and the opening and closing member 54 so that medicinal substance can be moved from the main tube 36 to the discharging tube 16. Unexplained reference numeral 62 indicates a block-detecting sensor for detecting blockage of the discharging tube 16, which will be described below.

Operation of the automatic dose infusion apparatus with the above-stated construction according to the preferred embodiment of the present invention will now be described in detail. First, a medicinal substance pack to be infused into a patient in the medicinal substance storage chamber 6 of the automatic dose infusion apparatus 2, and the hinged cover 22 of the driving chamber 12 is opened. The main tube 26 is mounted in the pressure pump 28 and engaged in the guide groove 32. The check valve 50 is securely located in the check valve location groove 34, and then the hinged cover 22 is closed.

After the hinged cover 22 is closed, the button unit 10 is manipulated to turn power on and set various doses and time. The set data is displayed on the LCD 11. Subsequently, the needle 20 is inserted into a prescribed region of a patient, and then a start button (not shown) of the button unit 10 is pushed so that infusion of the medicinal substance is initiated.

Since the hinged cover 22 is closed as described above, the supporting bar 24 protruded from the lower surface of the hinged cover 22 presses the upper part of the main tube 36 disposed in the pressure pump 28, and the pressing protrusion 26 formed at the lower surface of the hinged cover 22 adjacent to the supporting bar 24 presses the top of the opening and closing member 54 securely located in the check valve location groove 34. Consequently, the medicinal substance can be moved from the main tube 36 to the discharging tube 16.

When the patient pushes the start button, the motor is rotated, and the rotary driving force of the motor is transmitted to the cam 42 so that the cam 42 is rotated. As the cam 42 is rotated, the pressing pieces 30, through which the cam 42 is inserted longitudinally, are moved vertically cooperating with the cam 42.

The camshaft 44 and the motor shaft 38 are connected with each other by means of the spring 40. Consequently, the rotary driving force of the motor can be accurately transmitted to the cam 42 even when the camshaft 44 and the motor shaft 38 are not arranged in a straight line.

The bolus switch 14 is connected to the side of the housing 4 of the automatic dose infusion apparatus 2 via the electric cord. When the bolus switch 14 is manipulated, the number of rotations of the motor varies on the basis of a prescribed amount of medicinal substance. Preferably, the motor may be a DC motor or a linear motor, in which the rotary force of the motor is accurately controlled through the current control of the number of rotations of the motor.

The automatic dose infusion apparatus according to the preferred embodiment of the present invention with various sensors mounted thereto will be described below in detail with reference to the accompanying drawings.

The sensors mounted to the automatic dose infusion apparatus 2 according to the preferred embodiment of the present invention include a door-detecting sensor, an air-detecting sensor, and a block-detecting sensor.

Preferably, the door-detecting sensor, the air-detecting sensor, and the block-detecting sensor may be proximity sensors. When a subject is placed a prescribed distance from the proximity sensor, capacitance (i.e., electric capacity) between the subject and the proximity sensor changes. The proximity sensor detects whether the subject is present or not on the basis of a signal outputted in response to the change.

For example, materials having relatively high capacitance include liquid, metal, skin, etc. The proximity sensor outputs a high digital signal, i.e., "1" for the materials having relatively high capacitance. Materials having relatively low capacitance include air, plastic, etc. The proximity sensor outputs a low digital signal, i.e., "0" for the materials having relatively low capacitance. The proximity sensor has low power consumption. Consequently, the proximity sensor is suitable for a portable power supply.

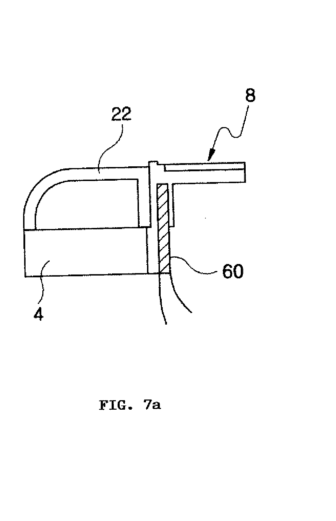

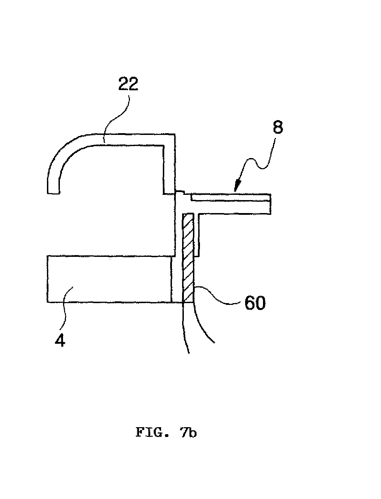

FIGS. 7a and 7b are side sectional views respectively showing a door-detecting sensor 60 of the automatic dose infusion apparatus according to the preferred embodiment of the present invention.

As shown in FIGS. 7a and 7b, the door-detecting sensor 60 of the automatic dose infusion apparatus 2 according to the preferred embodiment of the present invention is mounted to the inner wall of the housing 4 for detecting whether the hinged cover 22 (Refer to FIG. 2) is closed or not. When the hinged cover 22, which is made of a metallic material, is moved away from the door-detecting sensor 60, as shown in FIG. 7b, the door-detecting sensor 60 outputs a low signal, i.e., "0".

When the hinged cover 22 is moved toward the door-detecting sensor 60 as the hinged cover 22 is closed as shown in FIG. 7a, on the other hand, capacitance between the hinged cover 22 and the door-detecting sensor 60 changes. As a result, the door-detecting sensor 60 outputs a high signal, i.e., "1".

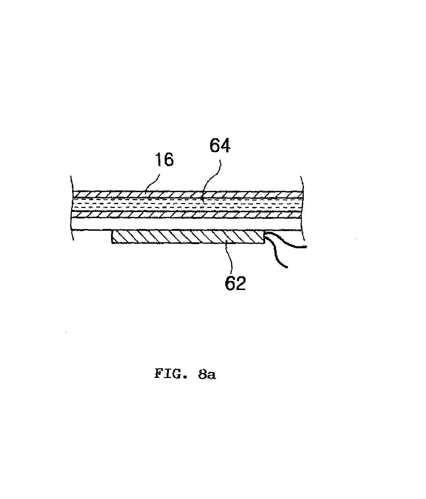

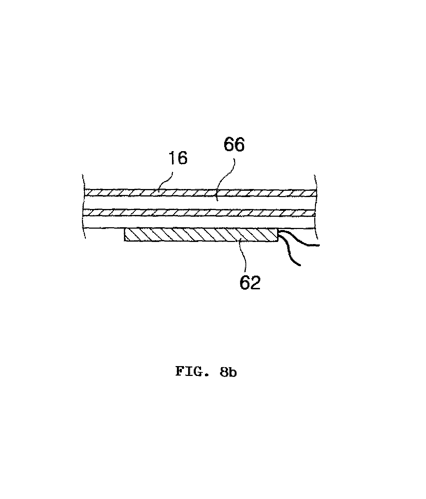

FIGS. 8a and 8b are side sectional views respectively showing an air-detecting sensor 62 of the automatic dose infusion apparatus according to the preferred embodiment of the present invention.

As shown in FIGS. 8a and 8b, the air-detecting sensor 62 of the automatic dose infusion apparatus 2 according to the preferred embodiment of the present invention is disposed at a prescribed position below the discharging tube 16. Preferably, the air-detecting sensor 62 may be mounted to the lower end of the guide groove, in which the discharging tube 16 is located, on the upper surface of the housing 4.

The air-detecting sensor 62 is disposed below the discharging tube 16 for detecting whether or not air 66 is contained in the medicinal substance passing through the discharging tube 16. When only medicinal substance passes through the discharging tube 16, the air-detecting sensor 62 outputs a high signal, i.e., "1".

When medicinal substance containing air therein passes through the discharging tube 16, on the other hand, the air-detecting sensor 62 outputs a low signal, i.e., "0".

If it is detected that air is contained in the discharging tube 16, a microprocessor (not shown) mounted in the control chamber 8 of the automatic dose infusion apparatus 2 stops the operation of the pressure pump 28 so that infusion of medicinal substance is interrupted.

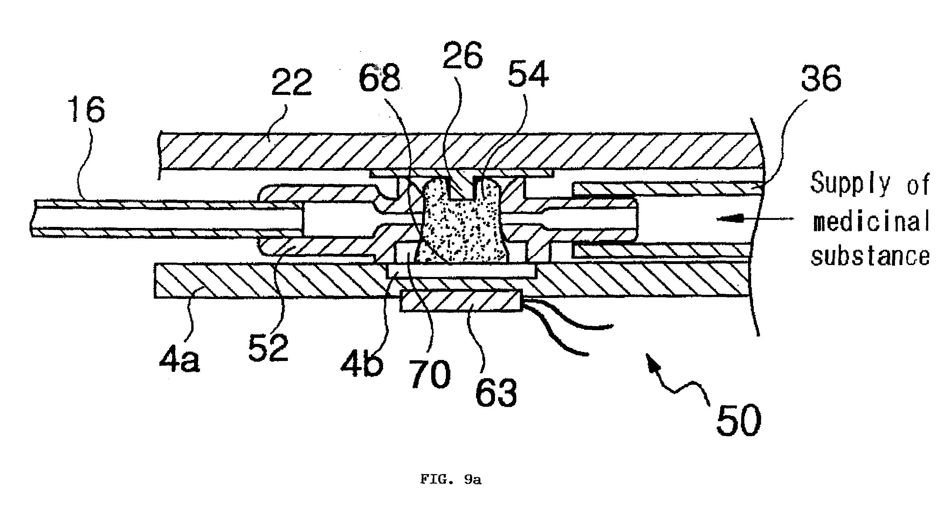

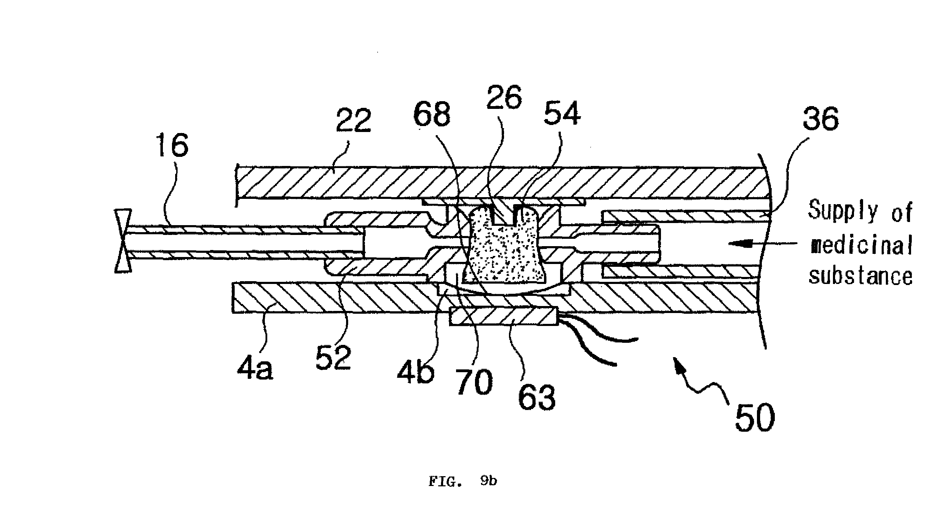

FIGS. 9a and 9b are side sectional views respectively showing a block-detecting sensor 63 of the automatic dose infusion apparatus according to the preferred embodiment of the present invention.

As shown in FIGS. 9a and 9b, the block-detecting sensor 63 of the automatic dose infusion apparatus 2 according to the preferred embodiment of the present invention is disposed below the regulating unit for regulating infusion of medicinal substance, i.e., the check valve. The check valve 50 includes a valve housing 52 for regulating flow of medicinal substance. The valve housing 52 has one end connected to the main tube 36 and the other end connected to the discharging tube 16. The valve housing 52 is pressed by means of the pressing protrusion 26 while the valve housing 52 is securely located in the check valve location groove 34 (Refer to FIG. 2). Through the valve housing 52 is formed a flow channel, which extends in the longitudinal direction of the housing 52.

The valve housing 52 has an inner middle space part 70 defined therein. One end of the flow channel of the valve housing 52 communicates with the main tube 36, and the other end of the flow channel of the valve housing 52 communicates with the discharging tube 36. The opening and closing member 54 formed of silicon packing is disposed in the inner middle space part 70 of the valve housing 52. The opening and closing member 54, which is formed in the shape of a taper, is moved vertically in the valve housing 52 to open or close the channel. Medicinal substance is introduced into the valve housing 52 through the main tube 36, passes below the opening and closing member 54, and is then discharged through the discharging tube 16. If the pressing protrusion 26 does not press the top of the opening and closing member 54 when medicinal substance is introduced into the valve housing 52 through the main tube 36 connected to a medicinal substance pack (not shown), the medicinal substance introduced into the valve housing 52 pushes the opening and closing member 54 upward to intercept the flow channel. Consequently, the medicinal substance is not moved from the main tube 36 to the discharging tube 16.

If the pressing protrusion 26 presses the top of the opening and closing member 54 as the hinged cover 4 is attached to the housing 4, on the other hand, the opening and closing member 54 is pressed downward. As a result, a gap is created between the flow channel of the valve housing 52 and the opening and closing member 54 so that medicinal substance can be moved from the main tube 36 to the discharging tube 16.

At a prescribed position of an upper supporting plate 4a of the housing 4, on which the check valve is securely located, is formed a hollow part 4b. The block-detecting sensor 63 is disposed below the hollow part 4b of the housing 4. Between the inner middle space part 70 and the hollow part 4b is disposed a membrane 68, by which the inner middle space part 70 and the hollow part 4b are isolated from each other.

When medicinal substance is moved from the main tube 36, which is connected to a medicinal substance pack (not shown), to the discharging tube 16 via the inner middle space part 70 of the valve housing 52, pressure in the inner middle space part 70 is normal. Consequently, the membrane 68 below the inner middle space part 70 is not deformed, as shown in FIG. 9a. At this time, the block-detecting sensor 63 outputs a low signal, i.e., "0".

When the patient-side tube, i.e., the discharging tube 16 is clogged or blocked as shown in FIG. 9b, pressure in the inner middle space part 70 of the valve housing 52 increases due to the medicinal substance filled in the inner middle space part 70. As the pressure in the inner middle space part 70 increases, the membrane 68 is expanded toward the hollow part 4b of the housing 4, which is disposed below the membrane 68. Consequently, the membrane 68 approaches the block-detecting sensor 63. At this time, the block-detecting sensor 63 outputs a high signal, i.e., "1".

In this case, a microprocessor (not shown) mounted in the control chamber 8 of the automatic dose infusion apparatus 2 stops the operation of the pressure pump 28 so that infusion of medicinal substance is interrupted, and sounds an alarm indicating that the discharging tube is blocked.

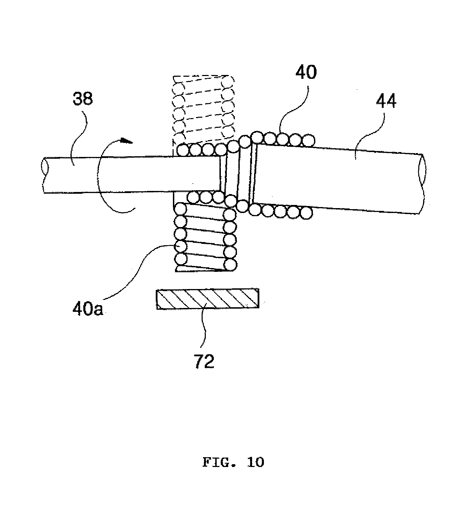

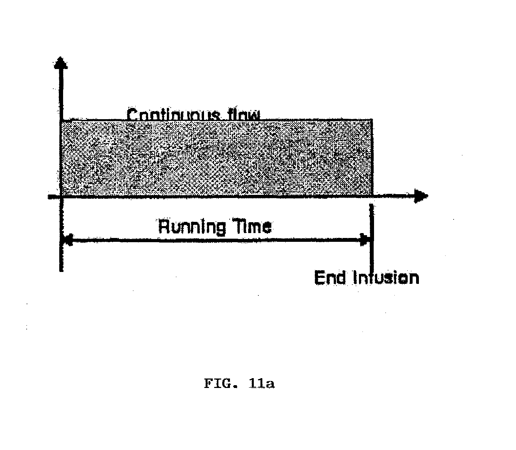

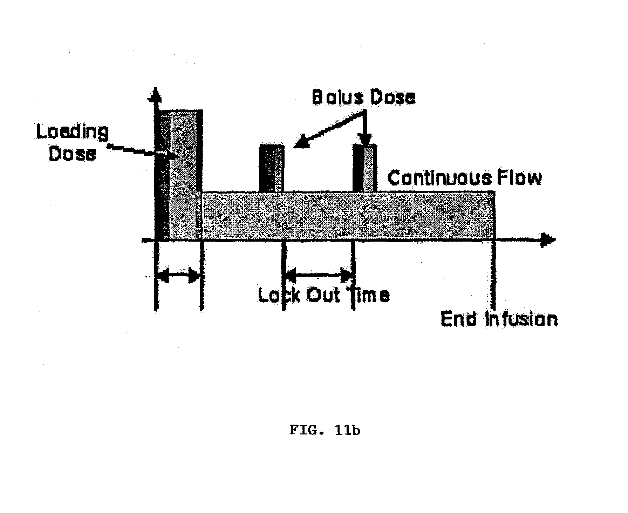

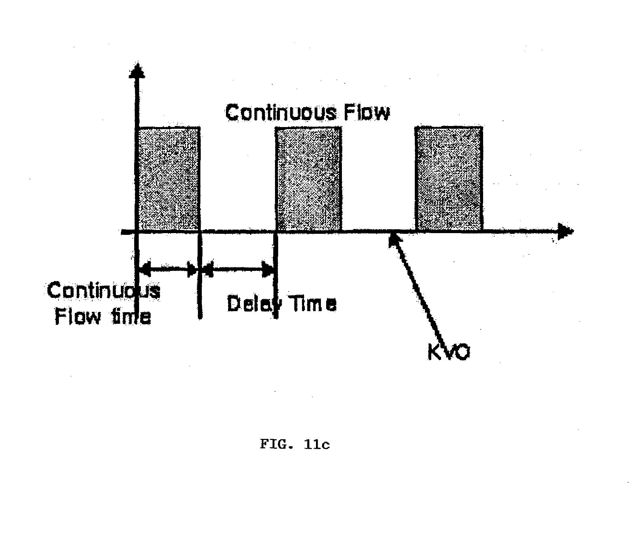

FIG. 10 is a side sectional view showing a rotation-detecting sensor 72 for detecting the number of rotations of a motor of the automatic dose infusion apparatus according to the preferred embodiment of the present invention.

As shown in FIG. 10, the rotation-detecting sensor 72 is mounted in the automatic dose infusion apparatus 2 according to the preferred embodiment of the present invention for detecting the number of rotations of a motor (not shown) that generates a driving force for driving the pressure pump 28.

The rotation speed of the motor, which is directly proportional to the infusion speed of the medicinal substance, is changed depending upon the number of rotations of the motor. Consequently, it is necessary to detect the number of rotations of the motor at regular intervals so that it is determined whether the motor is rotated at a prescribed speed. This is because the motor may be rotated at more than the prescribed speed as the motor is accelerated due to kinetic energy.

To this end, the camshaft 44 of the cam 42 mounted in the through-hole 30a formed in the pressing pieces 30 of the pressure pump 28 is connected to the motor shaft 38, by which a driving force is transmitted to the camshaft 44, by means of the spring 40 so that the rotary force of the motor can be accurately transmitted to the cam 42 even when the camshaft 44 and the motor shaft 38 are not arranged in a straight line.

At one end of the spring 40 is formed a protruded rotary part 40a, which extends from the spring 40 while being bent vertically from the spring 40. As the motor shaft 38 is rotated, the protruded rotary part 40a is also rotated about the same axis as the motor shaft 38. The rotation-detecting sensor 72 is disposed below the protruded rotary part 40a while being spaced a prescribed distance from the protruded rotary part 40a. To the protruded rotary part 40a is preferably attached a magnet. The rotation-detecting sensor 72 may be a hall sensor for detecting rotation of the motor shaft 38.

As the protruded rotary part 40a is rotated at the same rotating speed as the motor shaft 39, the protruded rotary part 40a is moved toward or away from the rotation-detecting sensor 72. As a result, the rotation-detecting sensor 72 outputs different output values.

For example, when the protruded rotary part 40a is moved toward the rotation-detecting sensor 72 as the motor shaft 38 is rotated, the rotation-detecting sensor 72 outputs a high signal, i.e., "1". When the protruded rotary part 40a is moved away from the rotation-detecting sensor 72 as the motor shaft 38 is rotated, on the other hand, the rotation-detecting sensor 72 outputs a low signal, i.e., "0".

In this case, a microprocessor (not shown) mounted in the control chamber 8 of the automatic dose infusion apparatus 2 operates signals transmitted from the rotation-detecting sensor 72 to determine whether the motor is being rotated at a speed corresponding to one of prescribed infusion speeds (i.e., high-speed infusion, intermediate-speed infusion, and low-speed infusion). If the rotating speed of the motor is not equal to the prescribed speed, microprocessor stops the operation of the pressure pump 28 so that infusion of medicinal substance is interrupted, and then corrects the error value to perform initialization.

Now, algorithms of the automatic dose infusion apparatus according to the preferred embodiment of the present invention will now be described in detail with reference to the accompanying drawings.

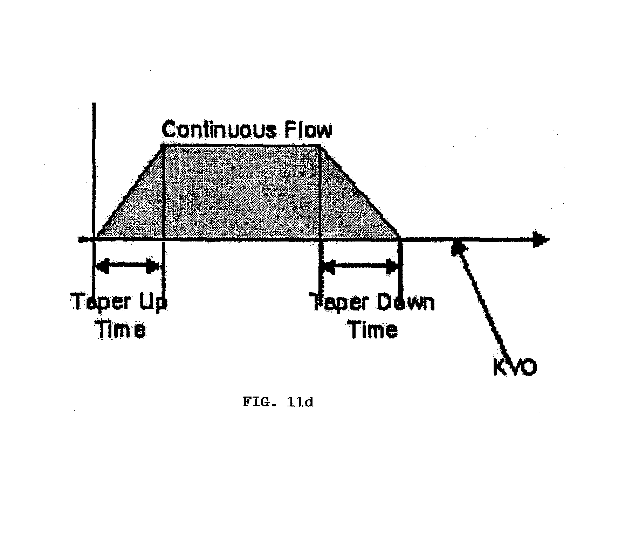

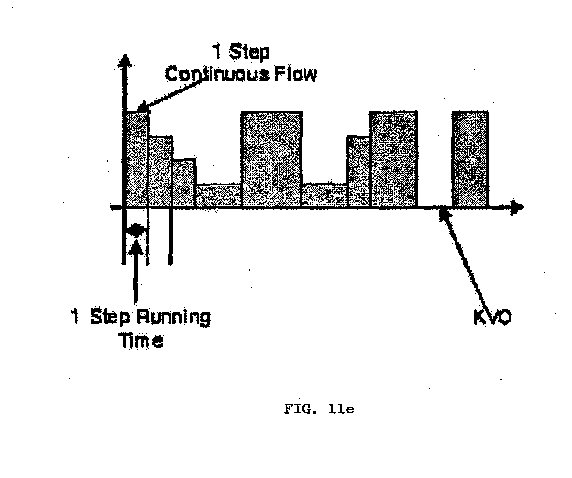

FIGS. 11a to 11e are graphs respectively showing time profiles at each mode of the automatic dose infusion apparatus according to the preferred embodiment of the present invention.

As shown in FIGS. 11a to 11e, medicinal substance infusion modes usable in the automatic dose infusion apparatus 2 of the present invention are classified into a continuous infusion mode, a patient controlled analgesia (PCA) mode, an intermittent infusion mode, a total parenteral nutrition (TPN) infusion mode, and a multi-step infusion mode.

The continuous infusion mode is used to continuously infuse a prescribed amount of medicinal substance from the time when infusion of the medicinal substance is initiated to the time when infusion of the medicinal substance is completed. The patient controlled analgesia (PCA) mode is used to further infuse another prescribed amount of medicinal substance in addition to the prescribed amount of the medicinal substance in the continuous infusion mode. After infusion of the medicinal substance is initiated, and then a prescribed amount of medicinal substance is infused for a prescribed period of time, the prescribed amount of medicinal substance is infused again for a prescribed period of infusion time. At this time, a patient may manipulate the bolus switch 14 to further infuse another prescribed amount of medicinal substance in addition to the prescribed amount of the medicinal substance in the continuous infusion mode. In the patient controlled analgesia (PCA) mode, the patient can control the self-infusion of the medicinal substance taking his/her pain into consideration.

According to the present invention, periods of infusion time and interruption time are preset from the time when infusion of the medicinal substance is initiated to the time when infusion of the medicinal substance is completed, and the infusion amount of the medicinal substance is also preset at each period of infusion time. The intermittent infusion mode is used to infuse the medicinal substance while alternately performing infusion and interruption of the medicinal substance.

The total parenteral nutrition (TPN) infusion mode is used to infuse the medicinal substance on the basis of three time zones. In a first time zone, the amount of the medicinal substance is gradually increased for a prescribed period of time after infusion of the medicinal substance is initiated. In a second time zone, a prescribed amount of medicinal substance is infused for a prescribed period of time. In a third time zone, the amount of the medicinal substance is gradually decreased for a prescribed period of time.

The multi-step infusion mode is used to infuse the medicinal substance, the amount of which is irregularly changed on the basis of multiple time zones. In the multi-step infusion mode, the irregular amount of the medicinal substance is infused to a patient who needs stimulation on nerves, brain, or muscles as the medicinal substance is infused.

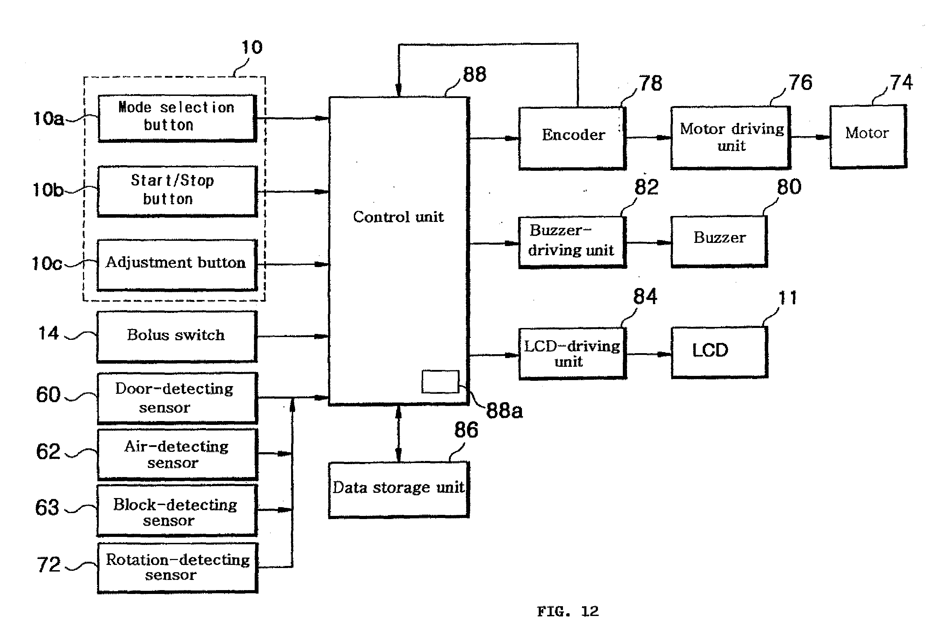

FIG. 12 is a block diagram showing the construction of the automatic dose infusion apparatus according to the preferred embodiment of the present invention.

As shown in FIG. 12, the automatic dose infusion apparatus 2 according to the preferred embodiment of the present invention includes a mode selection button 10a for selecting injection modes, a start/stop button 10b for starting or stopping infusion of the medicinal substance, and an adjustment button 10c for increasing or decreasing the infusion amount of the medicinal substance. Also, the automatic dose infusion apparatus 2 includes a bolus switch 14 for performing additional self-infusion of the medicinal substance.

The automatic dose infusion apparatus 2 according to the preferred embodiment of the present invention further includes a door-detecting sensor 60 for detecting whether the cover (not shown in FIG. 12) that securely locates the discharging tube 16 and accommodates the discharging tube 16 is closed or not, an air-detecting sensor 62 for detecting whether air is contained in the discharging tube 16 or not, a block-detecting sensor 63 for detecting whether the tube 16 is blocked or not, and a rotation-detecting sensor 72 for detecting the number of rotations of a motor 74, which operates a pressure pump (not shown in FIG. 12).

In addition, the automatic dose infusion apparatus 2 according to the preferred embodiment of the present invention includes a motor-driving unit 76 for driving the motor 74, and an encoder 78 for detecting the number of rotations of the motor 74. Also, the automatic dose infusion apparatus 2 according to the preferred embodiment of the present invention includes a buzzer 80 and a buzzer-driving unit 82. When the door is opened during infusion of the medicinal substance, when air is contained in the medicinal substance being infused, when a connection cable of the motor 74 is damaged or broken, when the motor is rotated under overload, when the discharging tube 16 is blocked, or when electric current of the battery is insufficient, the buzzer 80 is operated so that the patient can recognize an abnormality of the automatic dose infusion apparatus 2.

Information, such as the selected mode, the adjusted amount of the medicinal substance, and infusion start/stop status are displayed on an LCD 11 so that the patient can easily recognize the information. The LCD 11 is driven by means of an LCD-driving unit 84.

The automatic dose infusion apparatus 2 according to the preferred embodiment of the present invention includes a data storage unit 86 for storing medicinal substance infusion algorithms according to the continuous infusion mode, the patient controlled analgesia (PCA) mode, the intermittent infusion mode, the total parenteral nutrition (TPN) infusion mode, and the multi-step infusion mode as shown in FIGS. 11a to 11e, and data about the infusion amount of the medicinal substance according to the number of rotations of the motor.

The automatic dose infusion apparatus 2 according to the preferred embodiment of the present invention further includes a control unit 88 for receiving a signal according to the input of each button of the button unit 10 to perform an algorithm according to the corresponding mode stored in the data storage unit 86, detecting the number of rotations of the motor 74 in the infusion mode to control the operation of the motor 74, receiving the detected signal from each sensor to determine whether an abnormality is present or not, stopping the operation of the motor and operating the buzzer 80 when the abnormality is present, so that infusion of the medicinal substance can be safely and accurately performed. In the control unit 88 is mounted a timer 88a, by which a timing operation is performed so that the medicinal substance is infused while each mode is carried out according to the prescribed period of infusion time, which is set by a patient.

Operation of the automatic dose infusion apparatus with the above-stated construction according to the preferred embodiment of the present invention will now be described in detail with reference to the accompanying drawings.

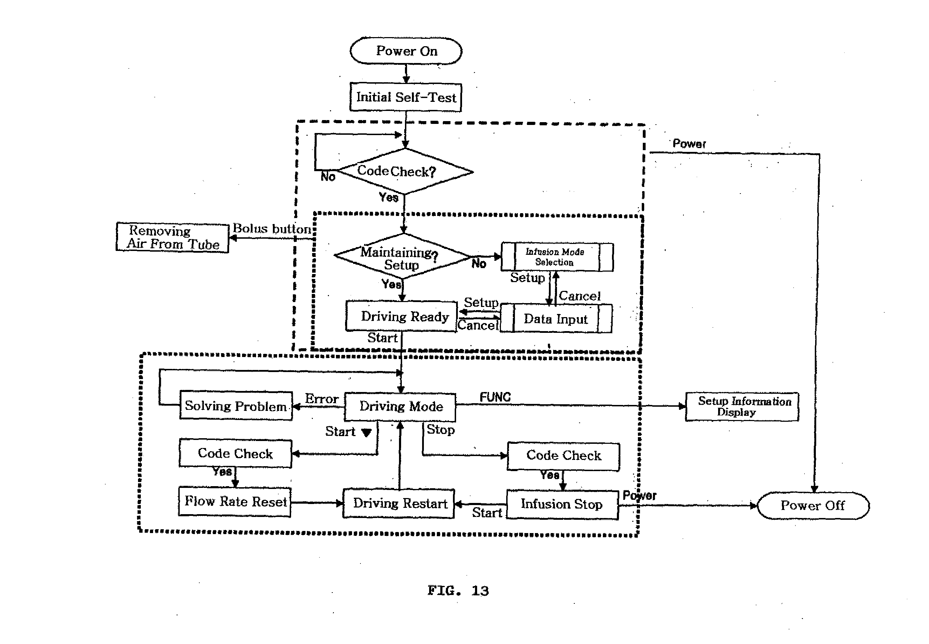

FIG. 13 is a flow chart showing all the operational algorithms of the automatic dose infusion apparatus according to the preferred embodiment of the present invention.

When electric current is supplied to the automatic dose infusion apparatus according to the preferred embodiment of the present invention, all data is initialized, and the control unit performs an initial self-diagnosis. In the initial self-diagnosis, status of the sensors and operation of the motor, buzzer, and the LCD are checked.

The control unit loads data of the automatic dose infusion apparatus, and checks a code. Under this condition, a user may select a hold mode for maintaining a previous setup or perform a new patient setup. When the user selects the hold mode, a driving ready state is achieved. When a new patient setup is necessary, the user selects an infusion mode. When the user selects the mode, all setup data (i.e., mode selection, the total infusion amount of medicinal substance, and period of infusion time) is set. After the setup is completed, a driving ready state is achieved. A check code may also be changed.

When the procedure proceeds to a driving mode from the driving ready state, the code may be checked on the basis of the user's determination, during the driving operation of the automatic dose infusion apparatus, to reset flow rate of the medicinal substance. At this time, new set values are stored in the data storage unit so that infusion of the medicinal substance is performed with the reset flow rate of the medicinal substance.

When an error is generated during the infusion of the medicinal substance, the control unit stops the operation of the motor to interrupt the infusion of the medicinal substance. After the problem is solved, the control unit starts infusion of the medicinal substance again. If the user manipulates the stop button to generate a stop signal, the control unit checks the corresponding code to stop the operation of the motor so that the infusion of the medicinal substance is stopped. And then, the power is turned off.

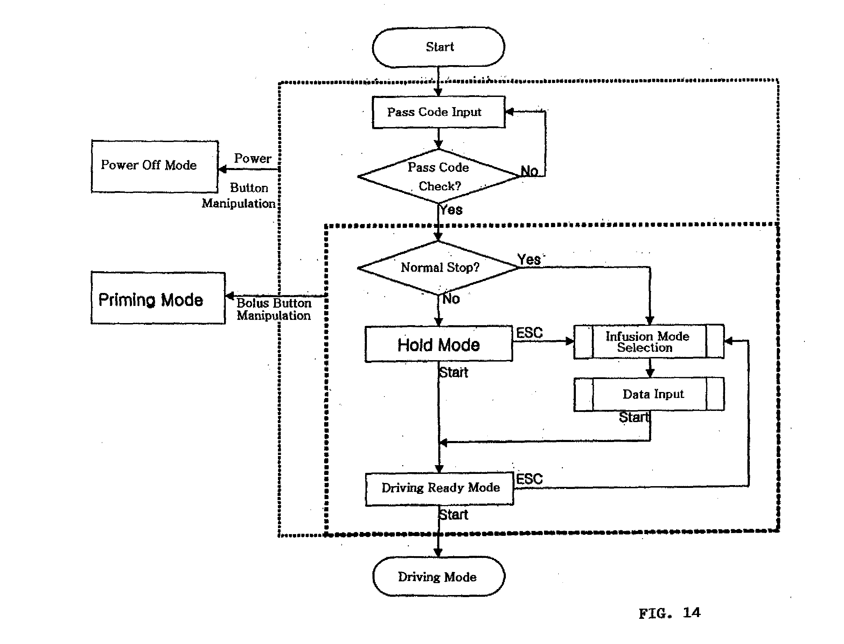

FIG. 14 is a flow chart showing a setup preparation mode algorithm of the automatic dose infusion apparatus according to the preferred embodiment of the present invention.

The automatic dose infusion apparatus according to the preferred embodiment of the present invention checks a pass code first when the power is turned on. Consequently, unauthorized access to the automatic dose infusion apparatus is not possible. After the pass code is checked, a user compares the set infusion amount of the medicinal substance to the infusion amount of the medicinal substance before the power is off to select a hold mode for maintaining a previous setup or an initial mode for performing a new setup.

In the hold mode, the infusion information before the power is off can be reviewed.

While a data setup preparation mode is being carried out, the control unit determines whether the bolus switch is manipulated or not. When the user manipulates the bolus switch, the control unit checks the door-detecting sensor and the pressure-detecting sensor. If the error is not generated, the control unit drives the motor. When the user does not manipulate the bolus switch, the control unit maintains a previous mode set before the bolus switch is manipulated.

When the user selects a previously set infusion mode, the control unit reads previously set infusion mode information from the data storage unit so that the user can immediately perform a driving ready mode.

When a new patient setup is necessary, the user selects an infusion mode to input necessary data.

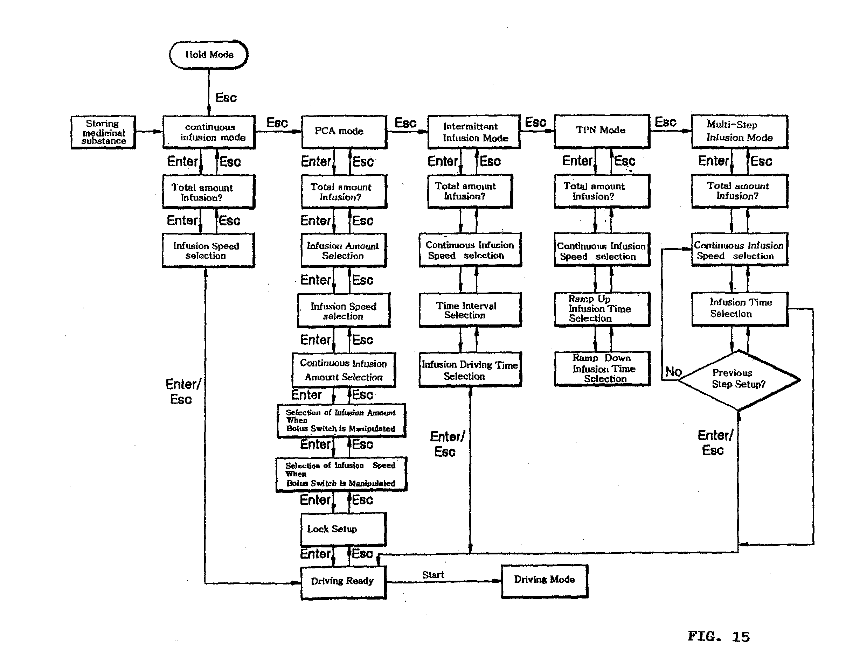

FIG. 15 is a flow chart showing a data selection setup algorithm of the automatic dose infusion apparatus according to the preferred embodiment of the present invention.

When a patient selects an infusion mode on the automatic dose infusion apparatus 2 according to the preferred embodiment of the present invention, it is necessary for him/her to select or input infusion modes as shown in FIG. 15. The infusion modes include the continuous infusion mode, the patient controlled analgesia (PCA) mode, the intermittent infusion mode, the total parenteral nutrition (TPN) infusion mode, and the multi-step infusion mode as shown in FIGS. 11a to 11e.

If the patient selects the continuous infusion mode, he/she can input the infusion amount of medicinal substance and the infusion speed of the medicinal substance. If the patient selects the total infusion amount of the medicinal substance or the infusion amount of the medicinal substance per unit time, and the infusion time of the medicinal substance, the infusion speed of the medicinal substance is decided. Consequently, it is possible for the patient to select the infusion amount of the medicinal substance and the infusion speed of the medicinal substance.

If the patient selects the patient controlled analgesia (PCA) mode, he/she can set the total infusion amount of the medicinal substance, the infusion speed of the medicinal substance, the continuous infusion amount of the medicinal substance, the continuous infusion speed of the medicinal substance, the infusion amount of the medicinal substance when the bolus switch is manipulated, the infusion speed of the medicinal substance when the bolus switch is manipulated, and a lock out time (a time interval between a first bolus switch manipulation and a second bolus switch manipulation).

If the patient selects the intermittent infusion mode, he/she can select the total infusion amount of the medicinal substance, the infusion speed of the medicinal substance, a time interval, and an infusion driving time.

If the patient selects the total parenteral nutrition (TPN) infusion mode, he/she can select the total infusion amount of the medicinal substance, the continuous infusion speed of the medicinal substance, a ramp up infusion time of the medicinal substance, and a ramp down infusion time of the medicinal substance.

If the patient selects the multi-step infusion mode, he/she can select the total infusion amount of the medicinal substance, the continuous infusion speed of the medicinal substance, and an infusion time of the medicinal substance. In this mode, the patient can set the previous step.

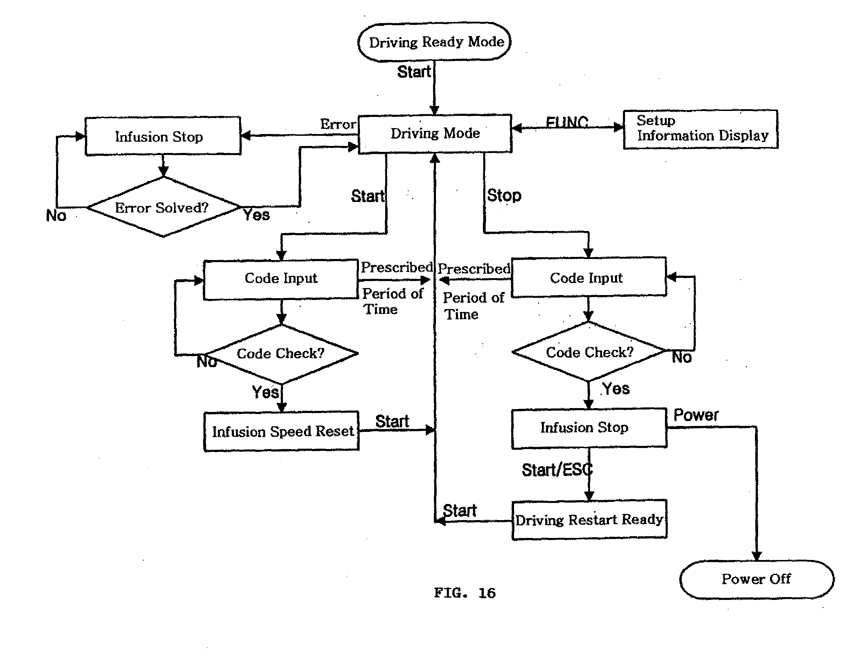

FIG. 16 is a flow chart showing key inputs for an infusion operation of the automatic dose infusion apparatus according to the preferred embodiment of the present invention.

When driving preparation of the automatic dose infusion apparatus 2 according to the preferred embodiment of the present invention is completed, the control unit 88 reads mode setup information from the data storage unit 86 to load the mode setup information, and checks an infusion start code and an infusion stop code at regular intervals. In other words, it is determined at regular intervals whether the infusion start code or the infusion stop code is generated from a key signal or a sensor detection signal.

If the infusion start code is generated, it is determined by the control unit whether the patient generates the key signal to reset the basic infusion speed of the medicinal substance. When the reset key signal is generated, the infusion speed of the medicinal substance is reset, and then a driving operation is carried out. If the infusion stop code is generated, on the other hand, the control unit reads the setup information to stop the infusion of the medicinal substance, and determines whether a power off key is inputted or another mode selection signal is generated. When another mode selection signal is generated, the control unit reads mode setup information to display it on a screen. When an error or the infusion stop signal is generated from the sensor while the infusion driving operation is being carried out, the control unit stops the infusion of the medicinal substance. After the problem is solved, the infusion driving operation is restarted.

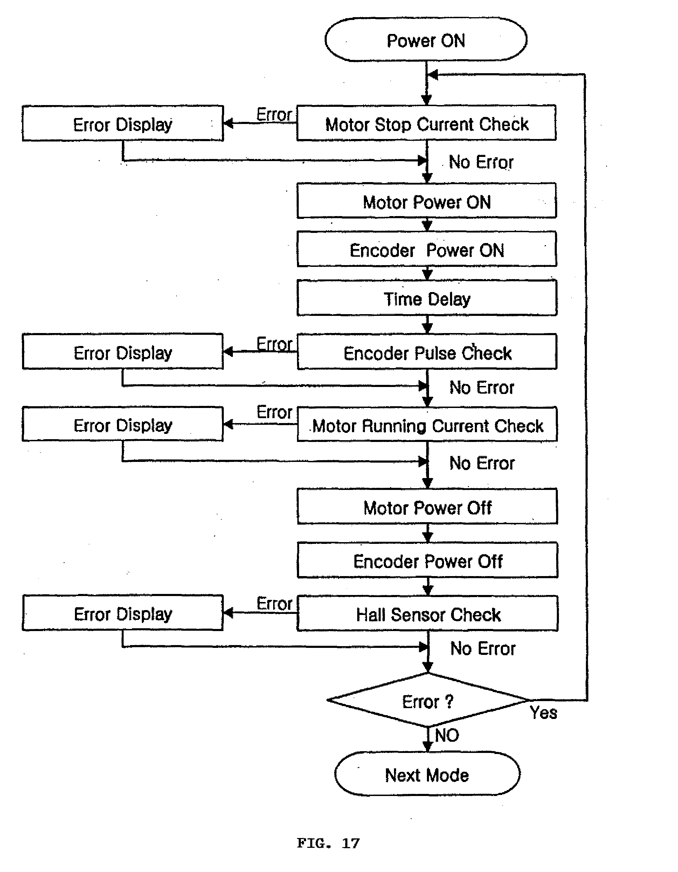

FIG. 17 is a flow chart showing a self-test algorithm of the automatic dose infusion apparatus according to the preferred embodiment of the present invention.

The self-test of the automatic dose infusion apparatus according to the preferred embodiment of the present invention is performed as follows: The current of the motor is checked during the operation of the motor. It is determined whether the motor is stopped or operated to check an error of the motor. If the error of the motor is not checked, the motor is operated.

While the motor is operated, a motor running current status, the operation of the encoder, and the coupling with the hall sensor are checked. If no error is generated, a next mode (code check) is performed.

When an error is generated during the self-test, the patient is informed of the error by means of a screen or an alarm. After a prescribed period of time expires, a retest process is carried out.

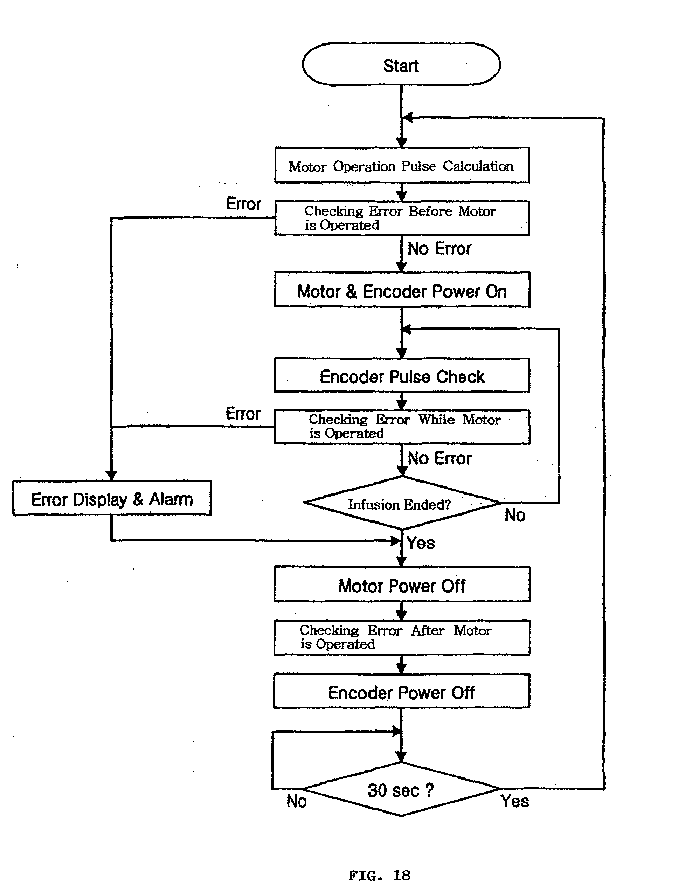

FIG. 18 is a flow chart showing an algorithm, when the motor is driven, of the automatic dose infusion apparatus according to the preferred embodiment of the present invention.

Before the motor of the automatic dose infusion apparatus according to the preferred embodiment of the present invention is driven, the number of pulses of the encoder per unit time is calculated on the basis of the current infusion speed of the medicinal substance. Also, the number of current operating pulses, to which increased or decreased rotation generated during the previous driving operation is compensated for, is calculated.

Before the motor is operated, an error of the motor is checked in the same manner as the self-test, and then electric current is supplied to the motor and the encoder so that the motor and the encoder can be operated. An error of the door-detecting sensor, the air-detecting sensor, or the pressure-detecting sensor is checked during the operation of the motor, and an error of the motor is checked in the same manner as the self-test. If the error is generated, the operation of the motor is immediately stopped.

The current pulse value of the encoder is compared to the prescribed pulse value of the encoder during the operation of the motor to stop the operation of the motor. At this time, the number of pulses of the encoder is checked to determine whether the rate of rotation of the motor is increased or decreased. After that, the encoder power is turned off so that electric energy consumption is reduced.

The above processes are continuously carried out at intervals of 30 seconds so that the prescribed amount of medicinal substance can be automatically infused.

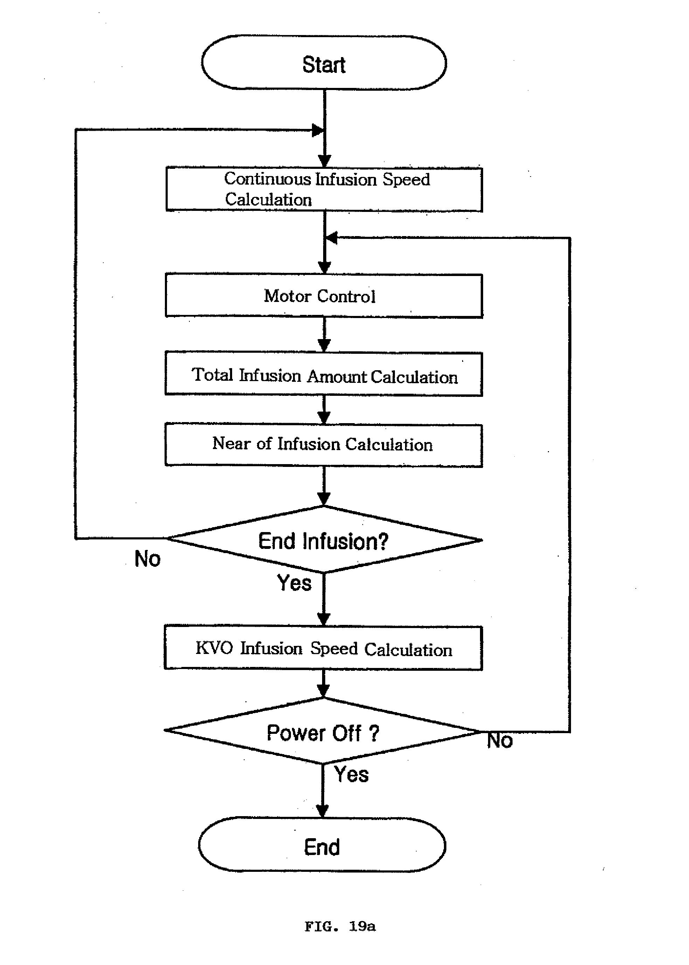

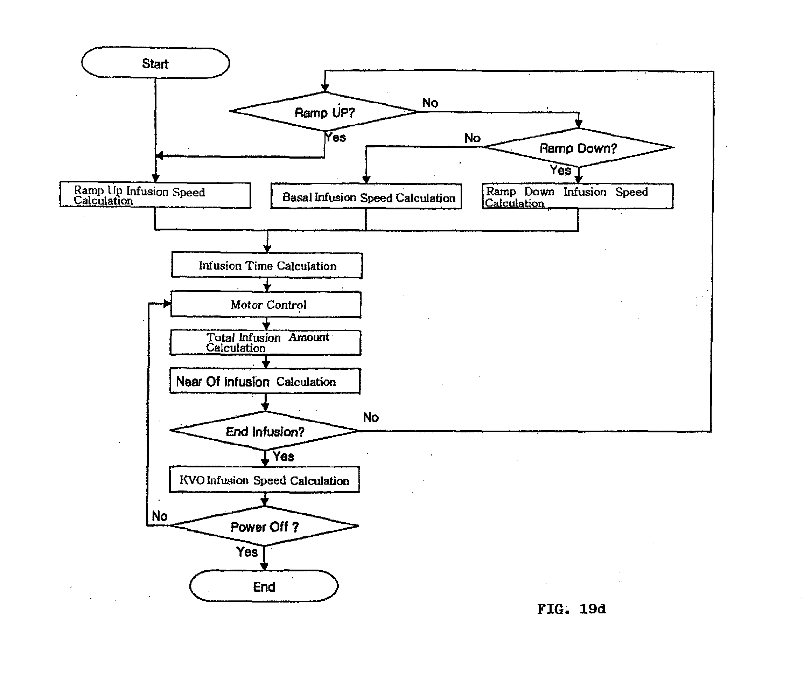

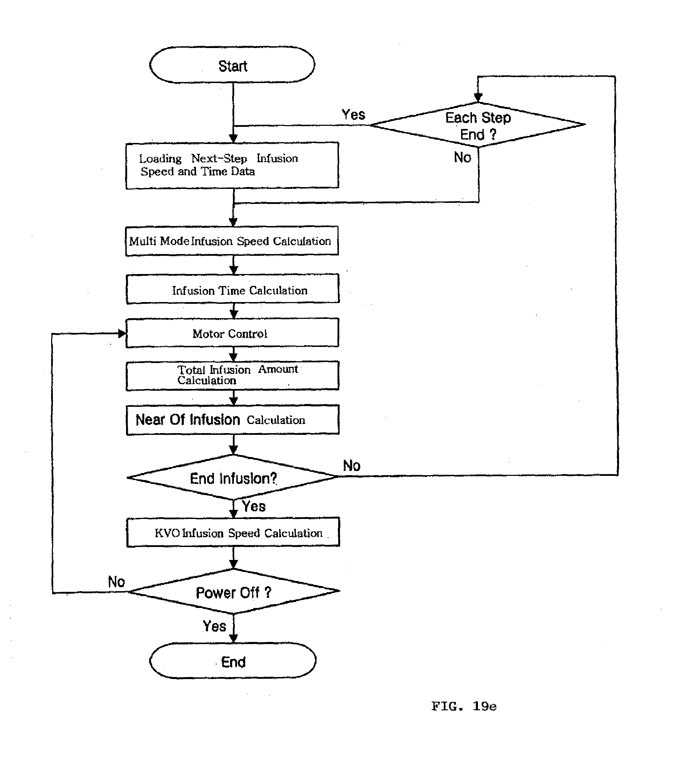

FIG. 19a is a flow chart showing an algorithm, in a continuous infusion mode, of the automatic dose infusion apparatus according to the preferred embodiment of the present invention.

The continuous infusion mode of the automatic dose infusion apparatus according to the preferred embodiment of the present invention is performed as follows: The motor running speed in the continuous infusion mode is calculated, and the motor is controlled in the same manner as in FIG. 18. The current infusion amount of the medicinal substance is calculated at every moment, the calculated infusion amount of the medicinal substance is compared to the prescribed infusion amount of the medicinal substance to check the completion of the infusion of the medicinal substance. Determination of whether the infusion of the medicinal substance is to be stopped in approximately 10 minutes is simulated through the use of the current infusion amount of the medicinal substance, and then the patient is informed of result of the simulation.

If the current infusion amount of the medicinal substance is more than the prescribed infusion amount of the medicinal substance, the infusion speed of the medicinal substance is converted into a KVO infusion speed, and then the power is ready to be turned off while the medicinal substance is infused in the same manner as described above.

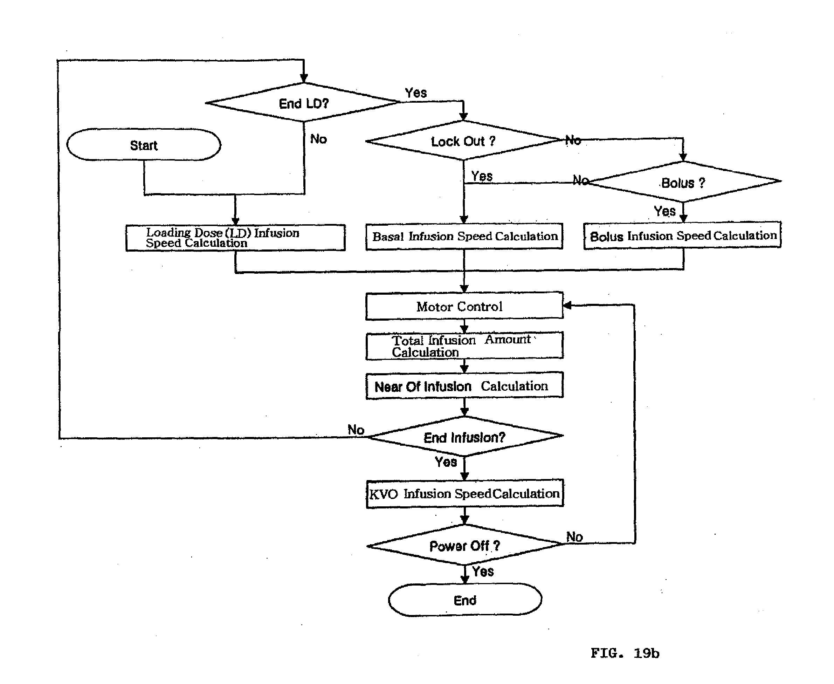

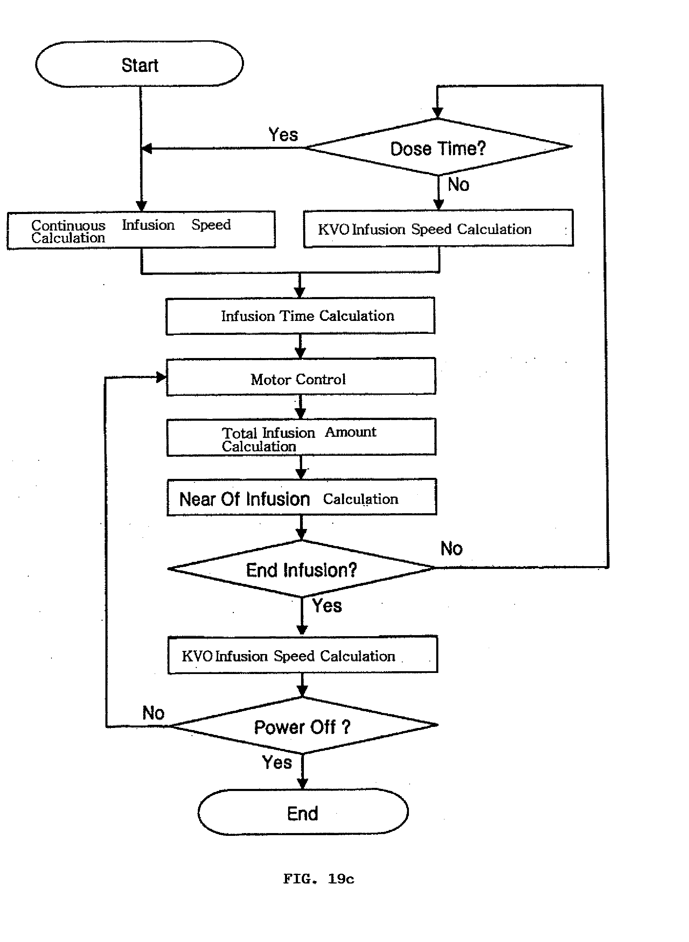

FIG. 19b is a flow chart showing an algorithm, in a patient controlled analgesia (PCA) mode, of the automatic dose infusion apparatus according to the preferred embodiment of the present invention.

The patient controlled analgesia (PCA) mode of the automatic dose infusion apparatus according to the preferred embodiment of the present invention is performed as follows: A loading dose of the medicinal substance is infused at the infusion speed of the medicinal substance in a loading dose infusion mode. When the infusion of the loading dose of the medicinal substance is completed, the medicinal substance is infused at a constant speed in a basal infusion mode. At this time, it is determined whether it is a lock out time or not. If it is the lock out time, the operation of the bolus switch is ignored. If it is the basal infusion mode, it is determined whether the bolus switch is operated or not to carry out the bolus infusion operation.

In the loading dose infusion mode and the basal infusion mode, the medicinal substance is infused by a unit time of 30 seconds. In the bolus infusion mode, on the other hand, the medicinal substance is infused by a unit time of 1 second.

The control unit of the automatic dose infusion apparatus calculates the rotating speed of the motor on the basis of each mode, and the motor is controlled in the same manner as in FIG. 18. The current infusion amount of the medicinal substance is calculated at every moment, the calculated infusion amount of the medicinal substance is compared to the prescribed infusion amount of the medicinal substance to check the completion of the infusion of the medicinal substance. Determination of whether the infusion of the medicinal substance is to be stopped in approximately 10 minutes is simulated through the use of the current infusion amount of the medicinal substance, and then the patient is informed of result of the simulation.

If the current infusion amount of the medicinal substance is more than the prescribed infusion amount of the medicinal substance, the infusion speed of the medicinal substance is converted into a KVO infusion speed, and then the power is ready to be turned off while the medicinal substance is infused in the same manner as described above.

FIG. 19c is a flow chart showing an algorithm, in an intermittent infusion mode, of the automatic dose infusion apparatus according to the preferred embodiment of the present invention.

The intermittent infusion mode of the automatic dose infusion apparatus according to the preferred embodiment of the present invention is performed as follows: It is determined whether the current mode is a medicinal substance infusion mode or a medicinal substance infusion stop mode. If the current mode is the medicinal substance infusion mode, the rotating speed of the motor in continuous infusion mode is calculated. If the current mode is the medicinal substance infusion stop mode, the rotating speed of the motor in the KVO infusion mode is calculated. The motor is controlled in the same manner as in FIG. 18. The current infusion amount of the medicinal substance is calculated at every moment, the calculated infusion amount of the medicinal substance is compared to the prescribed infusion amount of the medicinal substance to check the completion of the infusion of the medicinal substance. Determination of whether the infusion of the medicinal substance is to be stopped in approximately 10 minutes is simulated through the used of the current infusion amount of the medicinal substance, and then the patient is informed of result of the simulation.

If the current infusion amount of the medicinal substance is more than the prescribed infusion amount of the medicinal substance, the infusion speed of the medicinal substance is converted into a KVO infusion speed, and then the power is ready to be turned off while the medicinal substance is infused in the same manner as described above.

FIG. 19d is a flow chart showing an algorithm, in a total parenteral nutrition (TPN) infusion mode, of the automatic dose infusion apparatus according to the preferred embodiment of the present invention.

The total parenteral nutrition (TPN) infusion mode of the automatic dose infusion apparatus according to the preferred embodiment of the present invention is performed as follows: It is determined whether the current medicinal substance infusion mode is a ramp up mode or a ramp down mode, and the rotating speed of the motor is periodically calculated. If the current medicinal substance infusion mode is neither the ramp up mode nor the ramp down mode, the rotating speed of the motor in the basal infusion mode is calculated. The motor is controlled in the same manner as in FIG. 18. The current infusion amount of the medicinal substance is calculated at every moment, the calculated infusion amount of the medicinal substance is compared to the prescribed infusion amount of the medicinal substance to check the completion of the infusion of the medicinal substance. Determination of whether the infusion of the medicinal substance is to be stopped in approximately 10 minutes is simulated through the used of the current infusion amount of the medicinal substance, and then the patient is informed of result of the simulation.

If the current infusion amount of the medicinal substance is more than the prescribed infusion amount of the medicinal substance, the infusion speed of the medicinal substance is converted into a KVO infusion speed, and then the power is ready to be turned off while the medicinal substance is infused in the same manner as described above.

FIG. 19e is a flow chart showing an algorithm, in a multi-step infusion mode, of the automatic dose infusion apparatus according to the preferred embodiment of the present invention.

The multi-step infusion mode of the automatic dose infusion apparatus according to the preferred embodiment of the present invention is performed as follows: It is determined in which place the current medicinal substance infusion step is, and the rotating speed of the motor at the current step is calculated. The motor is controlled in the same manner as in FIG. 18. The current infusion amount of the medicinal substance is calculated at every moment, the calculated infusion amount of the medicinal substance is compared to the prescribed infusion amount of the medicinal substance to check the completion of the infusion of the medicinal substance. Determination of whether the infusion of the medicinal substance is to be stopped in approximately 10 minutes is simulated through the used of the current infusion amount of the medicinal substance, and then the patient is informed of result of the simulation.

If the current infusion amount of the medicinal substance is more than the prescribed infusion amount of the medicinal substance, the infusion speed of the medicinal substance is converted into a KVO infusion speed, and then the power is ready to be turned off while the medicinal substance is infused in the same manner as described above.

As apparent from the above description, the present invention provides a portable type automatic dose infusion apparatus that is capable of enabling a patient to perform self-administration of the prescribed amount of medicinal substance without regard to time and situation. The present invention also provides a method of controlling such an automatic dose infusion apparatus. Consequently, the present invention has the effect that the automatic dose infusion apparatus is very convenient to use. Especially, the patient can further self-inject an additional amount of medicinal substance simply by pushing a button whenever he/she feels pain. Also, the rotary force of a motor can be accurately transmitted even at a place where excessive vibration is generated through the use of a cam and a connecting member, such as a spring, whereby the prescribed amount of medicinal substance can be infused.

Furthermore, infusion of medicinal substance is controlled on the basis of signals detected by means of a block-detecting sensor for detecting blockage of a tube, a door-detecting sensor for detecting accurate accommodation of the tube, an air-detecting sensor for detecting air contained in the tube, and a rotation-detecting sensor for detecting the number of rotations of the motor. Consequently, the infusion of medicinal substance can be accurately and safely performed. Also, the number of rotations of the motor, on the basis of which the infusion speed of the medicinal substance is changed, can be checked, the infusion of medicinal substance can be stopped when an error is generated, the number of rotations of the motor can be compared to the prescribed value to compensate for the error, and an abnormality of the automatic dose infusion apparatus during the infusion of the medicinal substance can be checked at any time through the use of the above-mentioned various sensors.

Although the preferred embodiments of the present invention have been disclosed for illustrative purposes, those skilled in the art will appreciate that various modifications, additions and substitutions are possible, without departing from the scope and spirit of the invention as disclosed in the accompanying claims.

## Claims

1. An automatic dose infusion apparatus, comprising:
a housing having three compartments defined therein;
a medicinal substance storage chamber formed in the upper compartment of the housing for storing a medicinal substance pack;
a control chamber formed in the middle compartment of the housing while being disposed below the medicinal substance storage chamber, the control chamber including a button part for setting the infusion amount of medicinal substance and time intervals, an LCD for displaying setup information, and a circuit board with various circuit elements mounted thereto;
a driving chamber formed in the lower compartment of the housing while being disposed below the control chamber, the driving chamber including a pressure pump mounted therein for pumping the medicinal substance so that the medicinal substance can be discharged through a tube, the driving chamber serving to regulate the infusion of the medicinal substance by controlling the operation of the pressure pump;
a bolus switch connected to a prescribed position of one side of the housing via an electric cord for generating an additional medicinal substance infusion command signal; and
a tube extending from the inside of the driving chamber to the outside of the driving chamber for delivering the medicinal substance discharged from the medicinal substance pack to a needle.

2. The apparatus as set forth in claim 1, wherein the driving chamber includes:
a guide groove formed at the top of the housing for allowing the tube to be securely located therein;
a hinged cover attached to the top of the housing for covering the guide groove and the pressure pump; and
a check valve location groove formed at the top of the housing for allowing a check valve, formed at a prescribed position of the middle of the tube for regulating infusion of the medicinal substance, to be securely located therein.

3. The apparatus as set forth in claim 2, wherein the hinged cover includes:
a supporting bar formed at the lower surface of the hinged cover, such that the supporting bar extends a prescribed length in the longitudinal direction of the hinged cover, for pressing the tube against the pressure pump; and
a pressing protrusion for pressing the check valve so that the medicinal substance can be infused.

4. The apparatus as set forth in claim 2, wherein the pressure pump includes:
a plurality of pressing pieces disposed in a groove formed at the top of the housing, each of the pressing pieces being formed in the shape of a "U" such that the tube can be securely located in the upper part of the pressing piece and having a through-hole formed at the middle thereof;
a cam mounted in the through-holes of the pressing pieces, the cam having a screw formed on the outer circumference thereof;
a motor for transmitting a driving force to the cam; and
a spring for connecting a camshaft of the cam and a motor shaft of the motor such that the driving force of the motor can be accurately transmitted to the cam even when the camshaft and the motor shaft are not arranged in a straight line.

5. The apparatus as set forth in claim 4, wherein the pressing pieces are arranged such that one of the pressing pieces is connected to a neighboring pressing piece by means of the cam, the pressing pieces being moved vertically in the form of a sine wave to press the tube when the cam is driven so that the medicinal substance can be delivered.

6. The apparatus as set forth in claim 2, wherein the check valve comprises:
a valve housing having one end connected to a main tube for delivering the medicinal substance from the inside of the apparatus and the other end connected to a discharging tube for discharging the medicinal substance to the outside of the apparatus; and
an opening and closing member mounted in the valve housing such that the opening and closing member is disposed at a middle space part defined in the valve housing, the opening and closing member being formed in the shape of a taper so that the opening and closing member can be moved vertically to open or close a flow channel defined in the valve housing.

7. An automatic dose infusion apparatus, comprising:
a housing having three compartments defined therein;
a medicinal substance storage chamber formed in the upper compartment of the housing for storing a medicinal substance pack;
a driving chamber for regulating infusion of the medicinal substance, the driving chamber including a pressure pump mounted therein for pumping the medicinal substance so that the medicinal substance can be discharged through a tube;
a bolus switch connected to a prescribed position of one side of the housing via an electric cord for generating an additional medicinal substance infusion command signal;
a tube extending from the inside of the driving chamber to the outside of the driving chamber for delivering the medicinal substance discharged from the medicinal substance pack to a needle; and
a check valve including a valve housing having one end connected to one middle end of the tube and the other end connected to the other middle end of the tube, and an opening and closing member mounted in the valve housing such that the opening and closing member can be moved vertically to regulate infusion of the medicinal substance, wherein the automatic dose infusion apparatus further comprises:
a membrane attached to a lower supporting surface of the check valve such that the membrane can be expanded or contracted by the pressure inside the valve housing;
a block-detecting sensor disposed below the membrane for detecting blockage of the tube, the block-detecting sensor generating a high signal when the membrane is expanded;
a door-detecting sensor mounted to the inner wall of the housing for detecting whether the hinged cover is closed or not, the door-detecting sensor generating a high signal when the door is closed;
an air-detecting sensor disposed below the tube for detecting whether or not air is contained in the medicinal substance passing through the tube, the air-detecting sensor generating a high signal when only medicinal substance passes through the tube and the air-detecting sensor generating a low signal when medicinal substance containing air therein passes through the tube; and
a control unit for performing a control operation so that the operation of the pressure pump is stopped when a signal detected by means of the block-detecting sensor, the door-detecting sensor, or the air-detecting sensor is abnormal.

8. The apparatus as set forth in claim 7, wherein the block-detecting sensor, the door-detecting sensor, and the air-detecting sensor are proximity sensors each for generating a high signal when the sensor approaches liquid, metal, or skin.

9. The apparatus as set forth in claim 7, further comprising:
a rotation-detecting sensor mounted in the driving chamber for detecting the number of rotations of a motor that drives the pressure pump.

10. The apparatus as set forth in claim 9, further comprising: a spring connected to the motor, the spring being provided at one end thereof with a protruded rotary part, to which a magnet is attached, wherein the rotation-detecting sensor is a hall sensor for detecting rotation of a motor shaft of the motor.

11. An automatic dose infusion apparatus, comprising:
a button unit including a mode selection button for selecting an injection mode so that a prescribed amount of medicinal substance can be automatically infused into a patient, a start/stop button for starting or stopping infusion of the medicinal substance, and an adjustment button for increasing or decreasing the infusion amount of the medicinal substance;
a bolus switch for performing additional self-infusion of the medicinal substance in a patient controlled analgesia (PCA) mode;
a door-detecting sensor for detecting whether the cover that securely locates a tube and accommodates the tube is closed or not;
an air-detecting sensor for detecting whether air is contained in the tube or not;
a block-detecting sensor for detecting whether the tube is blocked or not;
a motor for actuating a pressure pump, and a motor-driving unit for driving the motor;
an LCD for displaying information, such as the mode selected by means of the button unit, the adjusted amount of the medicinal substance, and infusion start/stop status, so that the patient can easily recognize the information; and
an LCD-driving unit for driving the LCD, wherein the automatic dose infusion apparatus further comprises:
an encoder for detecting the number of rotations of the motor;
a buzzer for informing an abnormality of infusion of the medicinal substance, and a buzzer-driving unit for driving the buzzer;
a data storage unit for storing medicinal substance infusion algorithms according to a continuous infusion mode, a patient controlled analgesia (PCA) mode, an intermittent infusion mode, a total parenteral nutrition (TPN) infusion mode, and a multi-step infusion mode; and
a control unit for receiving a signal according to the input of each button of the button unit to perform an algorithm according to the corresponding mode previously stored in the data storage unit, detecting the number of rotations of the motor in the infusion mode to control the operation of the motor, receiving the detected signal from each sensor to determine whether an abnormality is present or not, stopping the operation of the motor and operating the buzzer when the abnormality is present, so that infusion of the medicinal substance can be safely and accurately performed.

12. A method of controlling an automatic dose infusion apparatus as set forth in any one of claims 1 to 11, comprising the steps of:
applying electric power to the automatic dose infusion apparatus;
performing an initial self-test;
performing an initial mode;
determining whether a prescribed mode is selected or not;
selecting an infusion mode;
preparing for the infusion mode;
performing infusion of medicinal substance;
informing that the infusion of medicinal substance will be stopped in approximately 10 minutes;
generating a stop code;
resetting flow rate of the medicinal substance during the operation of the automatic dose infusion apparatus;
checking an error in connection with the operation of a motor; and
stopping the infusion of the medicinal substance.

13. The method as set forth in claim 12, wherein the infusion mode comprises:
a continuous infusion mode for continuously infusing a prescribed amount of medicinal substance from the time when infusion of the medicinal substance is initiated to the time when infusion of the medicinal substance is completed;
a patient controlled analgesia (PCA) mode for further infusing another prescribed amount of medicinal substance in addition to the prescribed amount of the medicinal substance in the continuous infusion mode;
an intermittent infusion mode for presetting periods of infusion time and interruption time from the time when infusion of the medicinal substance is initiated to the time when infusion of the medicinal substance is completed, and resetting the infusion amount of the medicinal substance at each period of infusion time so that the infusion and the interruption of the medicinal substance can be alternately performed;
a total parenteral nutrition (TPN) infusion mode for gradually increasing the amount of the medicinal substance for a prescribed period of time, infusing a prescribed amount of the medicinal substance for a prescribed period of time, and gradually decreasing the amount of the medicinal substance for a prescribed period of time; and
a multi-step infusion mode for irregularly changing the amount of medicinal substance on the basis of multiple time zones so that the irregular amount of the medicinal substance can be infused on the basis of the multiple time zones.

14. The method as set forth in claim 12, wherein the initial mode performing step comprises:
determining whether the bolus switch is manipulated or not;
when the bolus switch is manipulated, checking a door-detecting sensor and a pressure-detecting sensor;
driving the motor; and
when the bolus switch is not manipulated, maintaining a previous mode set before the bolus switch is manipulated.

15. The method as set forth in claim 12, wherein the infusion mode selecting step comprises:
commonly selecting the total infusion amount of the medicinal substance, the infusion speed of the medicinal substance;
further selecting the infusion amount of the medicinal substance when the bolus switch is manipulated and a lock out time in the patient controlled analgesia mode;
further selecting an infusion time of the medicinal substance and an infusion stop time of the medicinal substance in the intermittent infusion mode;
further selecting a ramp up time of the medicinal substance, and a ramp down time of the medicinal substance in the total parenteral nutrition (TPN) infusion mode; and
further selecting an infusion step in the multi-step infusion mode.

16. The method as set forth in claim 12, wherein the infusion mode preparing step comprises:
checking an air-detecting sensor, a pressure-detecting sensor, and a door-detecting sensor;
determining whether an error is generated or not; and
generating a test alarm.

17. The method as set forth in claim 12, further comprising:
interrupting electric current supplied to an encoder and a hall sensor having no connection with the operation of the motor a prescribed period of time after the operation of the motor is stopped so that electric energy consumption of a battery is reduced.

18. The method as set forth in claim 12, wherein the medicinal substance infusion performing step comprises:
driving the motor on the basis of an ON/OFF control, a PWN control, or a PID control;
detecting the number of pulses of the motor with an encoder and a hall sensor when the motor is rotated to compare the detected number of pulses of the motor to a prescribed number of rotations of the motor; and
compensating for the detected number of pulses of the motor until the detected number of pulses of the motor is equal to the prescribed number of rotations of the motor when the number of rotations of the motor is increased or decreased.
